# EUROPEAN PATENT SPECIFICATION

(11) **EP 1 687 024 B1**
(45) Date of publication and mention of the grant of the patent: **19.10.2011**
(21) Application number: 04818605.0
(22) Date of filing: 27.10.2004
(51) Int. Cl.: A61K 39/385, A61K 47/48, A61K 31/70

(54) **CARBOHYDRATE ANTIGEN-NANOPARTICLE CONJUGATES AND USES THEREOF AS ANTIMETASTATIC AGENTS IN TREATING CANCER**
KOHLENHYDRAT-ANTIGEN-NANOTEILCHEN-KONJUGATE UND IHRE VERWENDUNGEN ALS ANTIMETASTATISCHE MITTEL BEI DER BEHANDLUNG VON KREBS
CONJUGUES DE NANOPARTICULES-ANTIGENES CARBOHYDRATE ET UTILISATION COMME AGENTS ANTI-METASTATIQUES DANS LE TRAITEMENT DU CANCER

(30) Priority: 05.11.2003 WO PCT/US03/34897; 22.03.2004 US 554994 P
(43) Date of publication of application: 09.08.2006
(73) Proprietor: THE GOVERNMENT OF THE UNITED STATES OF AMERICA, AS REPRESENTED BY THE SECRETARY, DEPARTMENT OF HEALTH AND HUMAN SERVICES, Rockville, MD 20852-3804 (US); The Research Foundation of State University of New York, Amherst, NY 14228 (US)
(72) Inventor: BARCHI, Jr., Joseph., J., Frederick, MD 21702 (US); RITTENHOUSE-OLSON, Kate, Williamsville, NY 14221 (US); SVAROVSKY, Sergei, Frederick, MD 21702 (US)
(74) Representative: Hallybone, Huw George
(86) International application number: PCT/US2004/035831
(87) International publication number: WO 2005/046722

(56) References cited:
- WO-A-02/32404
- BARCHI JOSEPH J JR ET AL: "Synthesis and properties of carbohydrate- and glycopeptide-bearing nanoparticles." ABSTRACTS OF PAPERS AMERICAN CHEMICAL SOCIETY, vol. 226, no. 1-2, 2003, page CARB 40, XP009047320 & 226TH ACS (AMERICAN CHEMICAL SOCIETY) NATIONAL MEETING; NEW YORK, NY, USA; SEPTEMBER 07-11, 2003 ISSN: 0065-7727
- HAKOMORI S: "ABERRANT GLYCOSYLATION IN TUMORS AND TUMOR-ASSOCIATED CARBOHYDRATE ANTIGENS" ADVANCES IN CANCER RESEARCH, ACADEMIC PRESS, LONDON, GB, vol. 52, 1989, pages 257-331, XP002905215 ISSN: 0065-230X
- GLINSKY VLADISLAV V ET AL: "Intravascular metastatic cancer cell homotypic aggregation at the sites of primary attachment to the endothelium." CANCER RESEARCH, vol. 63, no. 13, 1 July 2003 (2003-07-01), pages 3805-3811, XP002327998 ISSN: 0008-5472 cited in the application
- GLINSKY VLADISLAV V ET AL: "The role of Thomsen-Friedenreich antigen in adhesion of human breast and prostate cancer cells to the endothelium" CANCER RESEARCH, vol. 61, no. 12, 15 June 2001 (2001-06-15), pages 4851-4857, XP002327999 ISSN: 0008-5472
- KHALDOYANIDI SOPHIA K ET AL: "MDA-MB-435 human breast carcinoma cell homo- and heterotypic adhesion under flow conditions is mediated in part by Thomsen-Friedenreich antigen-galectin-3 interactions." JOURNAL OF BIOLOGICAL CHEMISTRY, vol. 278, no. 6, 7 February 2003 (2003-02-07), pages 4127-4134, XP002328000 ISSN: 0021-9258
- RAGUPATHI GOVINDASWAMI ET AL: "Vaccines prepared with sialyl-Tn and sialyl-Tn trimers using the 4-(4-maleimidomethyl)cyclohexane-1-carboxy l hydrazide linker group result in optimal antibody titers against ovine submaxillary mucin and sialyl-Tn-positive tumor cells" CANCER IMMUNOLOGY IMMUNOTHERAPY, vol. 48, no. 1, April 1999 (1999-04), pages 1-8, XP002328001 ISSN: 0340-7004
- LLOYD K O: "Carbohydrate vaccines for the immunotherapy of cancer" DRUG NEWS AND PERSPECTIVES 2000 SPAIN, vol. 13, no. 8, 2000, pages 463-470, XP001206504 ISSN: 0214-0934
- SVAROVSKY S A ET AL: "Synthesis of gold nanoparticles bearing the Thomsen-Friedenreich disaccharide: a new multivalent presentation of an important tumor antigen" TETRAHEDRON: ASYMMETRY, PERGAMON, OXFORD, GB, vol. 16, no. 2, 24 January 2005 (2005-01-24), pages 587-598, XP004743805 ISSN: 0957-4166

## Description

### FIELD OF THE INVENTION

The present invention is in the field of bionanotechnology. More specifically, the present invention pertains to carbohydrate nanoparticle conjugates. The present invention also pertains to methods of synthesizing carbohydrate nanoparticle conjugates. The present invention also pertains to methods of reducing metastasis of carcinoma cells using carbohydrate nanoparticle conjugates.

### BACKGROUND OF THE INVENTION

Nearly all living organisms have carbohydrates covalently linked to proteins or lipids on the surfaces of its cells. Many such carbohydrates have been shown to mediate many important physiological phenomena such as cell-cell/cell-matrix communication and signal transduction. As a result, cell surface glycans are thought to play important roles in the pathology of many diseases, *e.g.*, inflammation, restenosis, bacterial/viral infections and cancer. An established hallmark of tumorigenesis is the biosynthesis of aberrant glycan chains due to changes in the expression of glycoprocessing enzymes. These aberrations are believed to become more marked as a tumor acquires a more aggressive phenotype. Many of these glycans are known tumor-associated antigens that have been used as haptens in the development of tumor vaccines.

Two of the most widely distributed cancer-associated cell surface carbohydrate moieties is the tumor-associated Thomsen-Friedenreich ("T" or "TF" or "T_{F}") disaccharide antigen (Gal-β-1→3-GalNAc-α1-O-Ser/Thr and its corresponding "TN" or "Tₙ" or "Tn" or "T nouvelle" monosaccharide antigen (GalNAc-α1-O-Ser/Thr). TF and Tn antigens are classic carbohydrate antigens that are shielded in healthy and benign-diseased tissues, but are uncovered in about 90% of carcinomas (*e.g*., breast, colon, prostate, bladder, ovarian, and gastric). Tn and T epitopes are cell and tissue adhesion molecules, operative in invasion by and metastasis of carcinoma which includes adherent and proliferative phases. Springer, G. F., J. Mol. Med., 1997 Aug;75(8):594-602. For example, during metastasis, it is believed that cancer cells released from a primary tumor to the circulatory system secrete TF antigen bearing glycoproteins. These TF glycoproteins are believed to induce endothelial cells to express the cell surface protein galectin-3, which gives rise to initial binding of the released cancer cells to the endothelial cells. Subsequent protein-protein interactions between the cancer cells and endothelial cells are believed to be mediated by integrin, which gives rise to colonization of the metastasized cancer cells. Accordingly, disruption of the TF antigen-mediated, tumor-endothelial cell interactions using synthetic compounds that either mimic or mask the carbohydrate structure have been highly sought after. Glinksy, V. V., et al., Cancer Res., 2001, 61, 4851-4857. In this regard, there is a continuing need to develop synthetic compounds that inhibit tumor cell adhesion for use in antiadhesive cancer therapeutics.

U.S. Patent Publication No. 2004/0052729A1 discloses materials and methods for studying and modulating the interaction of carbohydrate-containing moieties with other species, *e.g*., clusters of metal or semiconductor atoms, which can be employed as a substrate for immobilizing a plurality of ligands comprising carbohydrate groups. Although this patent application publication describes several carbohydrate-coated gold nanoparticles, the disclosed carbohydrates (*e.g*., lactose) are not believed to be a specific tumor associated carbohydrate antigens that are expressed (*i.e*., displayed) on tumor surfaces. Thus, there is a continuing need to develop synthetic compounds having specific tumor associated carbohydrate antigens that are expressed on the surface of tumor cells.

### SUMMARY OF THE INVENTION

In overcoming the problems associated with providing synthetic compounds that inhibit tumor cell adhesion for use in antiadhesive cancer therapeutics, one aspect of the present invention provides, *inter alia,* methods of preparing antigen-nanoparticle conjugates, comprising providing a nanoparticle and conjugating a plurality of carbohydrate antigens to the nanoparticle, wherein the carbohydrate antigens include TF antigen.

Another aspect of the present invention provides antigen-nanoparticle conjugate comprising a plurality of carbohydrate antigens conjugated to a nanoparticle, wherein the antigens include TF antigen.

Other aspects of the present invention include the use of antigen nanoparticle conjugates of the invention for the manufacture of a medicament for inhibiting metastasis of carcinoma cells in a mammal. For example, methods of synthesizing conjugated nanoparticles include linking a plurality of TF antigens to the surfaces of gold nanoparticles. These nanoparticles have been injected into mammals bearing breast tumors and this treatment has been shown to preventmetastasis to the lungs.

### BRIEF DESCRIPTION OF THE DRAWINGS

The foregoing summary, as well as the following detailed description, is further understood when read in conjunction with the appended drawings. For the purpose of illustrating the invention, there is shown in the drawings exemplary embodiments of the invention; however, the invention is not limited to the specific methods, compositions, and devices disclosed. In the drawings:

**Fig 1** (a) Structure of Thomsen Friedenreich antigen, and (b) Structure of Tn antigen. Lines from the amino and acid functions designate extension of a protein chain.

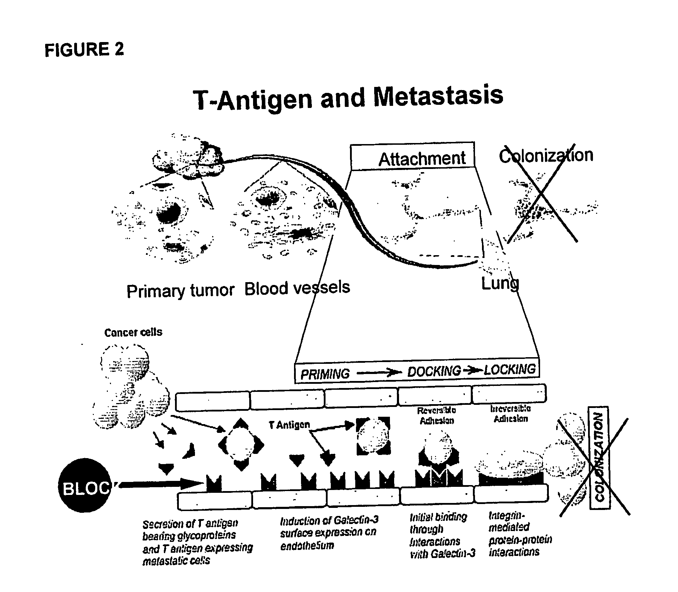

**Fig 2****.** Putative involvement of T-antigen in tumor metastasis from Glinskii, Cancer Res., 2001, 61, p4851.

**Fig 3****.** Transmission Electron micrograph of T-ag coated gold Nanoparticles

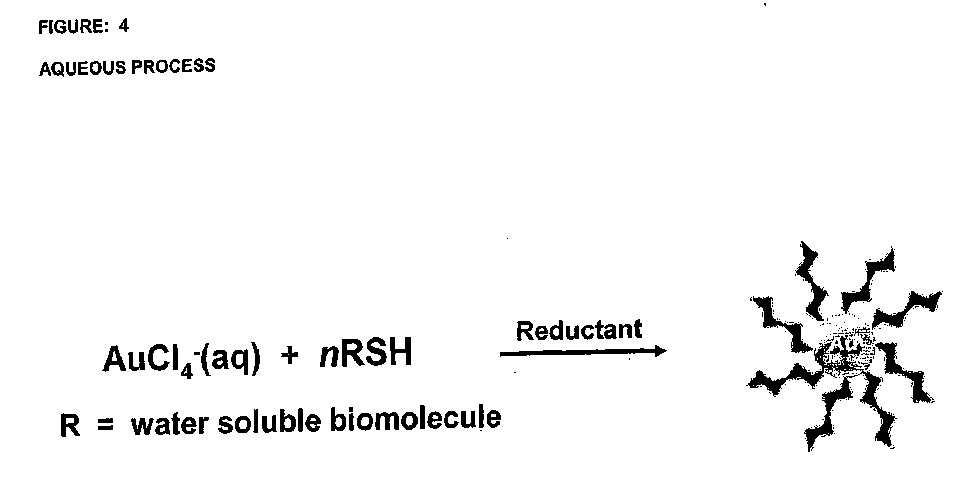

**Fig 4****.** One step procedure for the preparation of gold nanoparticles in aqueous solution.

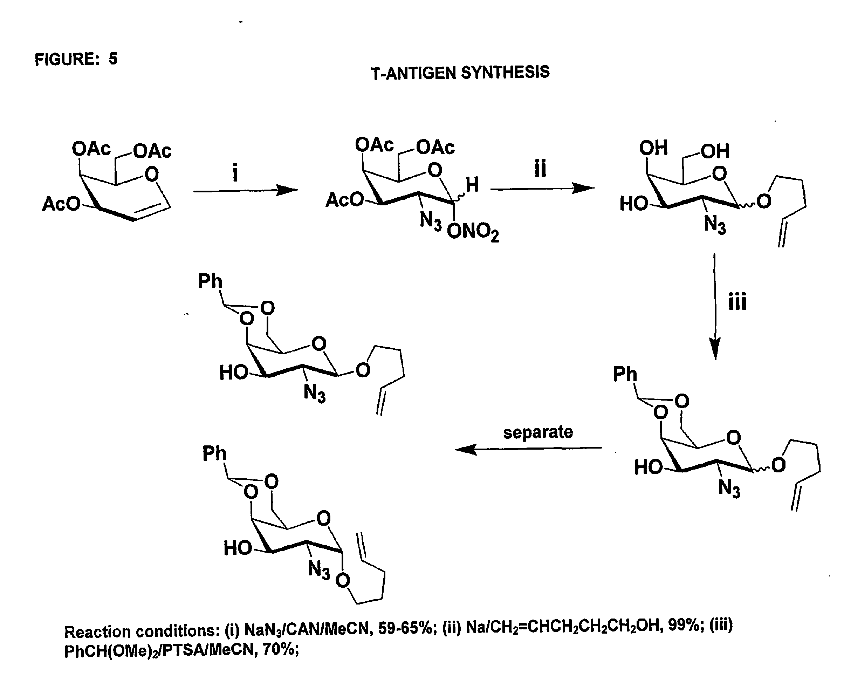

**Fig 5****.** Synthesis of Tn building block for Au nanoparticle preparation. Conditions are shown. Adapted from Carbohydr. Res. 2003, 338, 1925-1935.

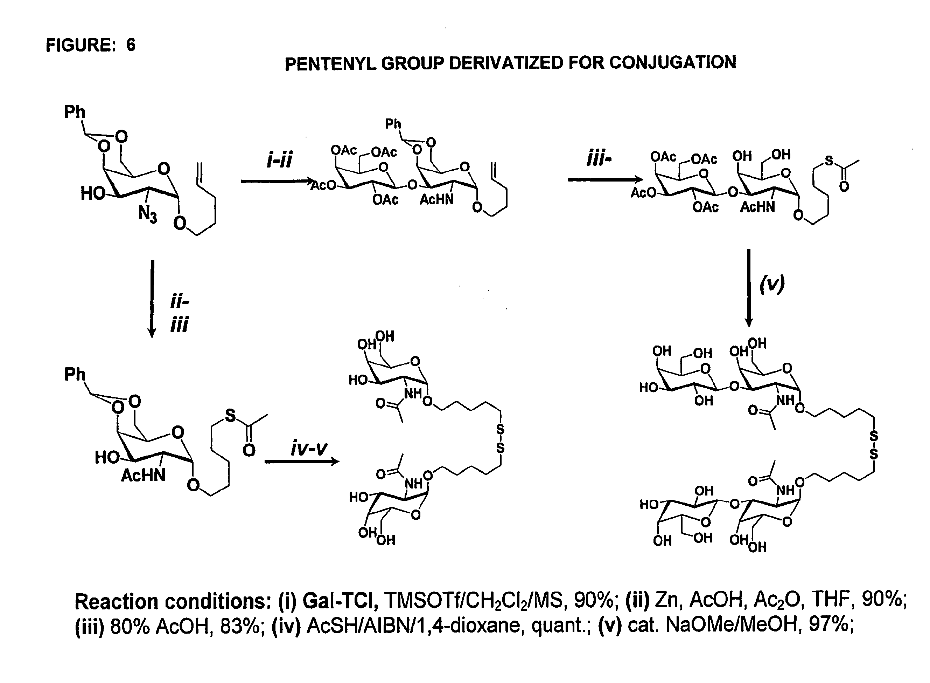

**Fig 6****.** Synthesis of TF building block for Au nanoparticle preparation. Conditions are shown. Adapted from Carbohydr. Res. 2003, 338, 1925-1935.

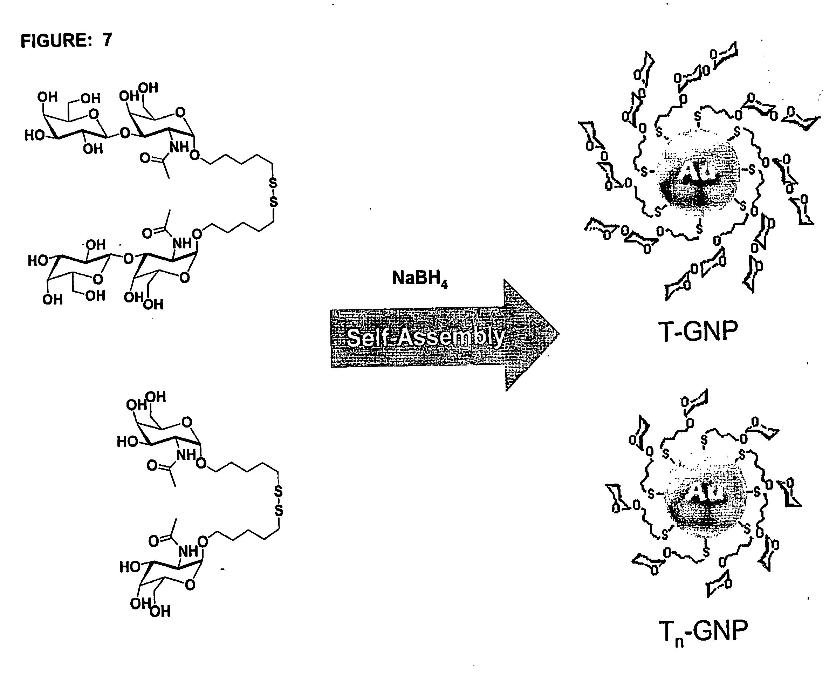

**Fig 7****.** Synthesis of TF and Tn antigen-coated Au nanoparticles.

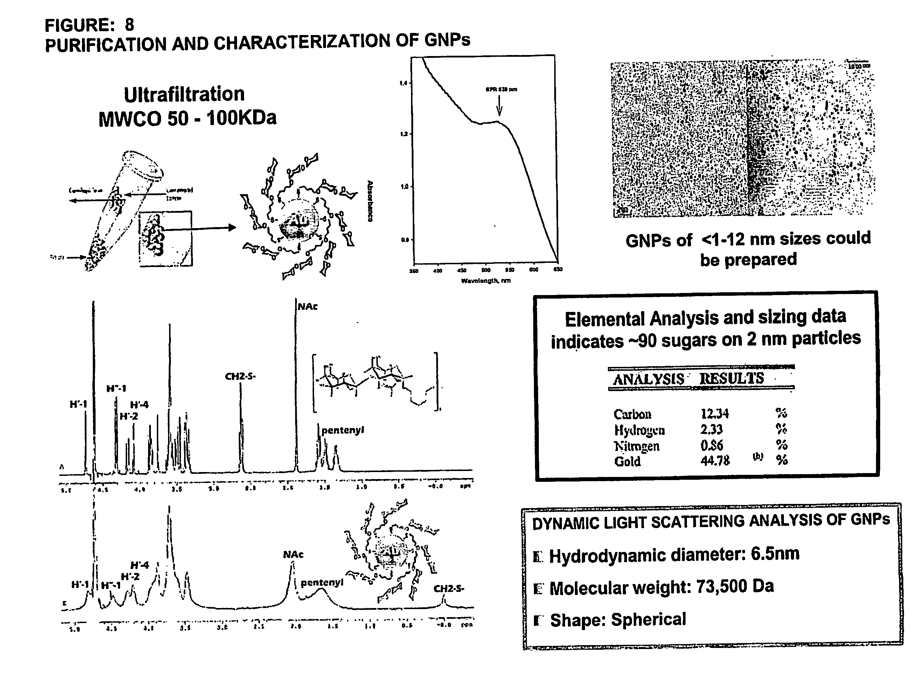

**Fig 8****.** Characterization of TF-coated gold nanoparticles. Shown are data from TEM, NMR, ultrafiltration and elemental analysis.

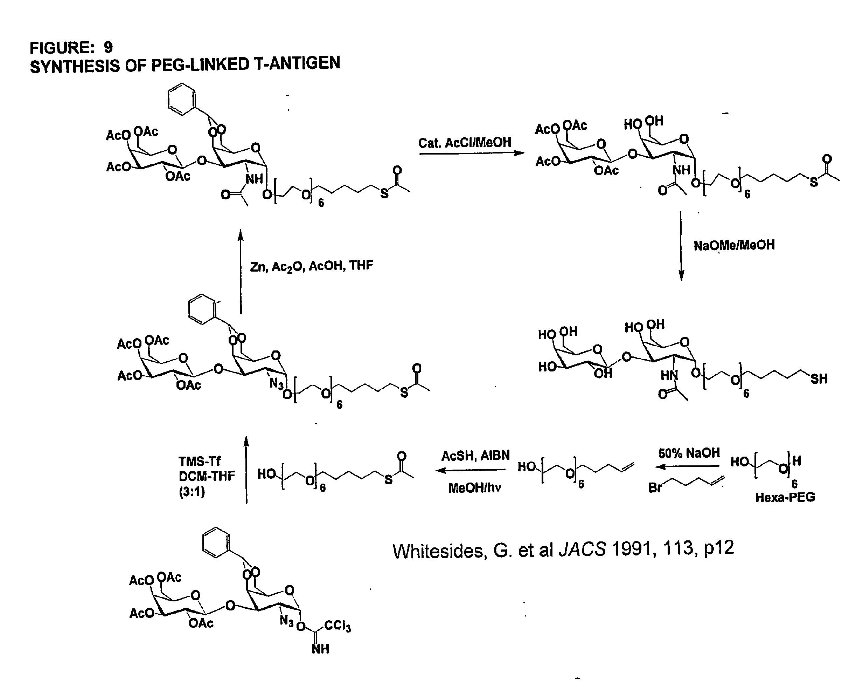

**Fig 9****.** Synthesis of a specific PEG-linked TF antigen building block for carbohydrate nanoparticle synthesis.

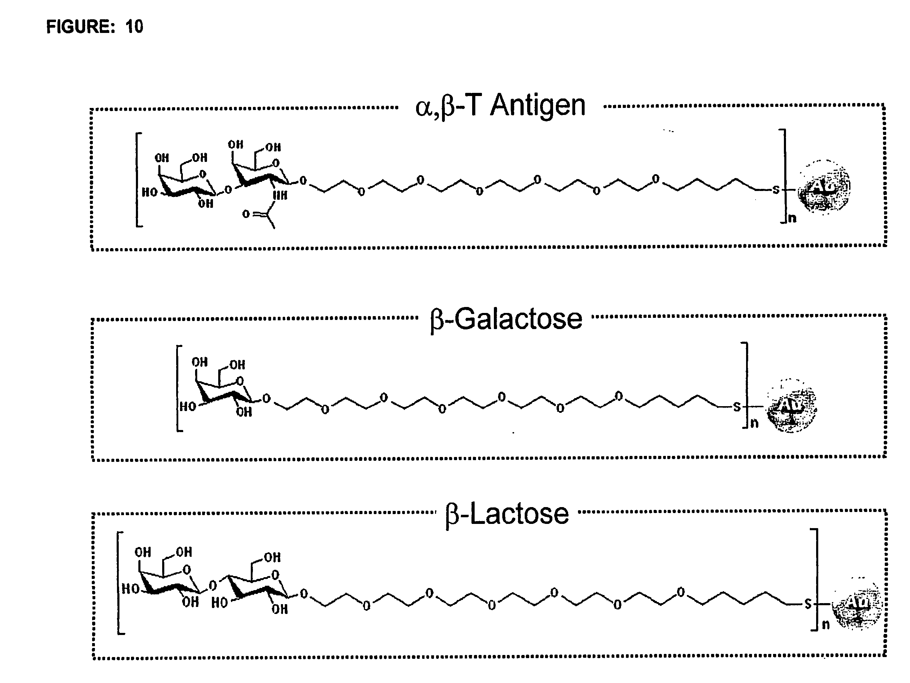

**Fig 10****.** Additional sugar-coated nanoparticles prepared in the study.

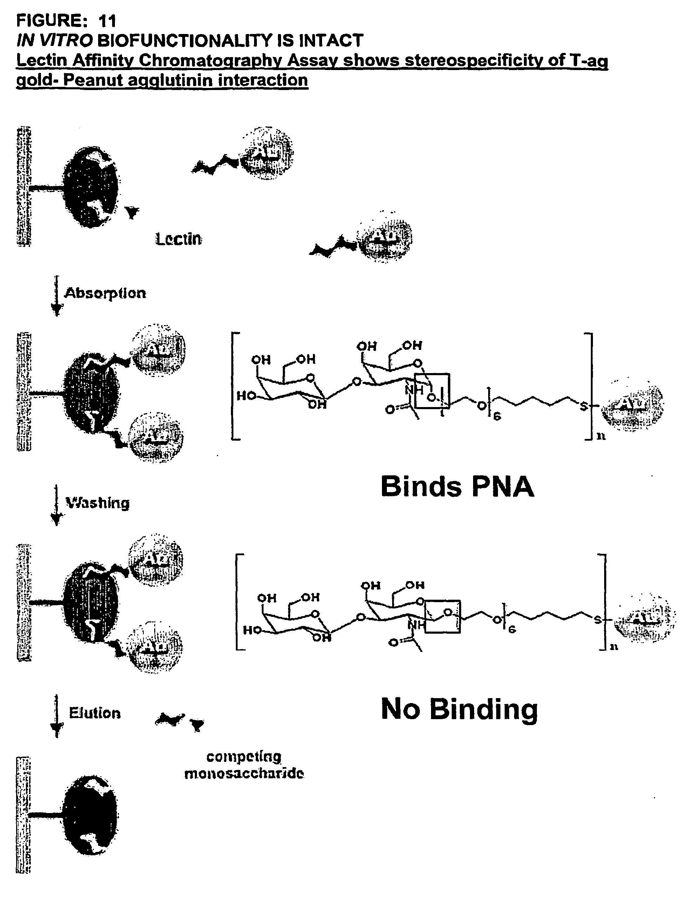

**Fig 11****.** Schematic of the lectin affinity chromatography assay which proves the stereospecificity of T-ag gold's interaction with Peanut agglutinin (PNA).

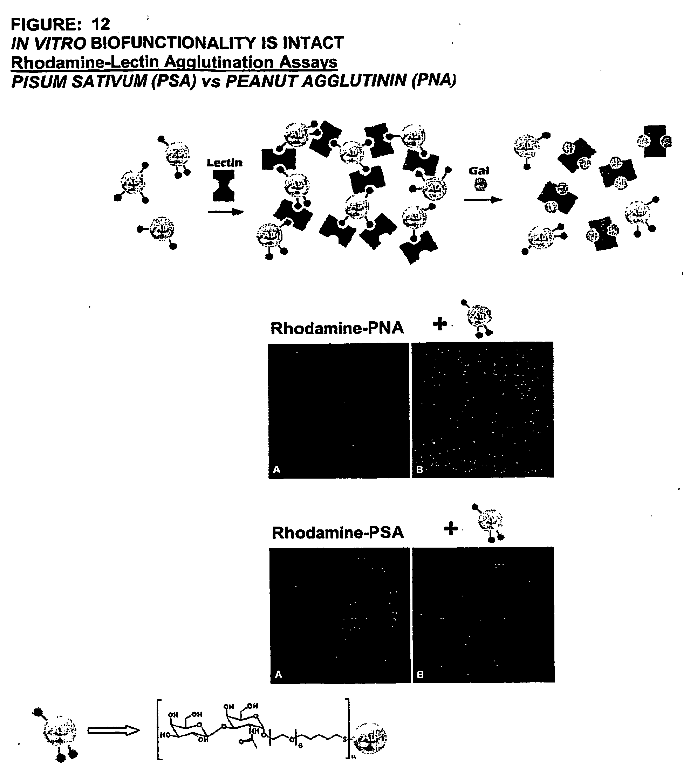

**Fig 12****.** Comparison of binding of T-ag gold particles to rhodamine-conjugated peanut agglutinin or Pisum sativum lectin. The T-ag particles form clear aggregates with the PNA-rhodamine but not with PSA rhodamine.

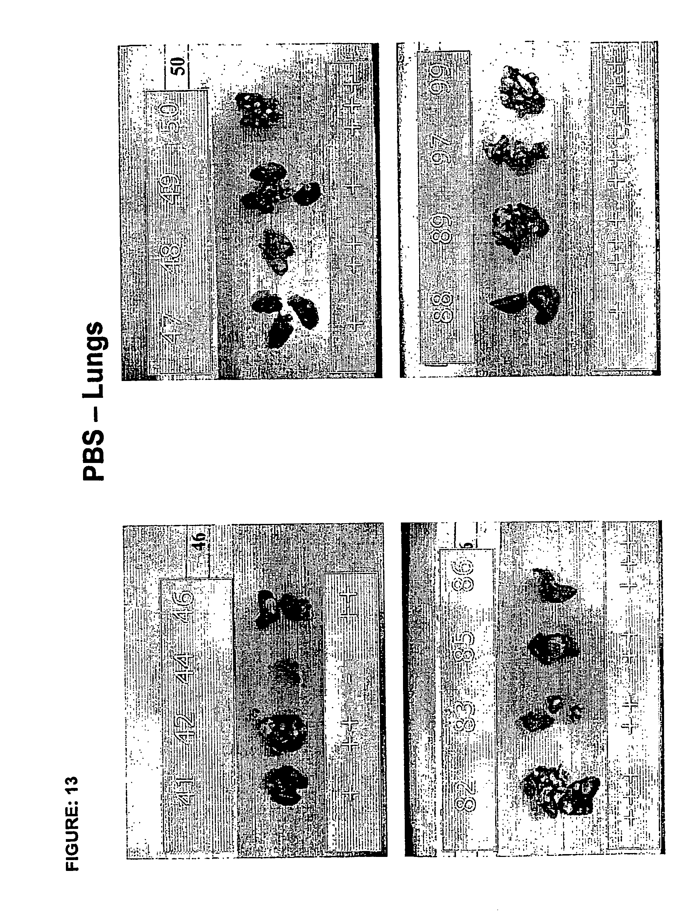

**Fig. 13****.** Lungs from PBS Group. Number of metastatic lesions grossly visibly counted, +++>20, ++ (10-20), + (1-9), - (0).

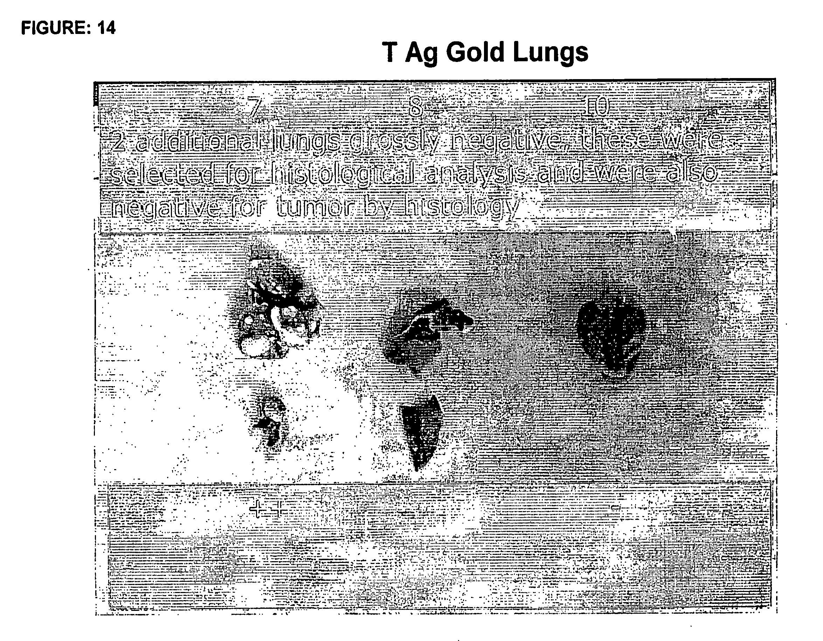

**Fig. 14****.** Lungs from T Ag Group. Number of metastatic lesions grossly visibly counted, +++>20, ++ (10-20), + (1-9), - (0).

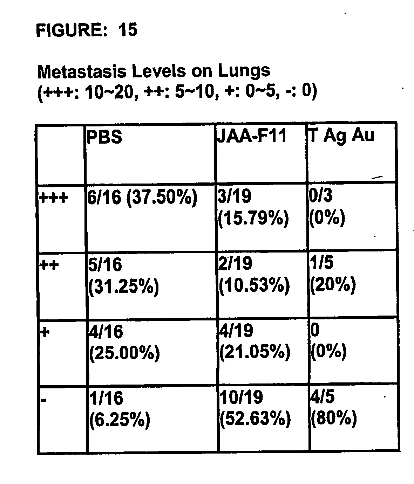

**Fig 15****.** Table summarizing the levels of metastases on lungs.

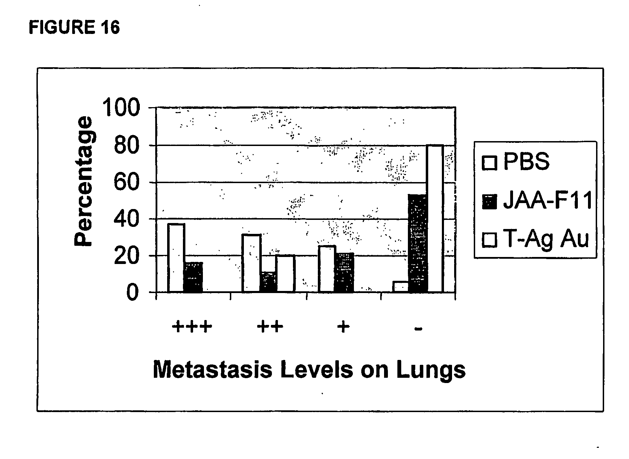

**Fig 16****.** Metastasis rates presented as a bar graph, PBS, JAA-F11 treated and TAg gold treated groups+++>20, ++ (10-20), + (1-9), - (0).

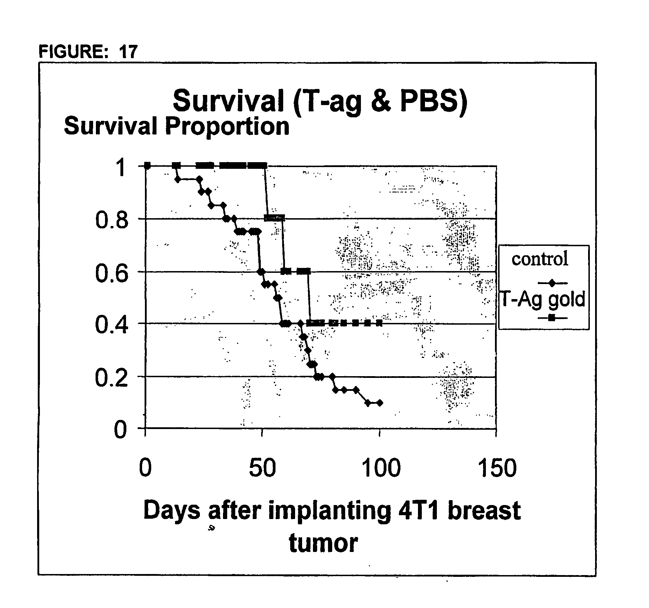

**Fig. 17****.** Kaplan-Meier Curve of *in vivo* Experiment. (Group 1: PBS control; Group 2: T Ag gold treatment).

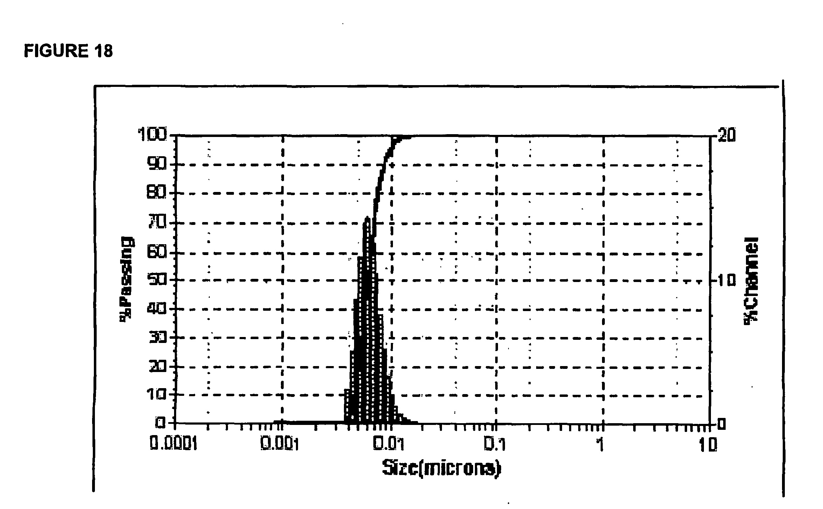

**Fig.18****.** Particle size distribution results for sample TF-HEXPEG-AuNP.

### DETAILED DESCRIPTION OF ILLUSTRATIVE EMBODIMENTS

The present invention may be understood more readily by reference to the following detailed description of the invention taken in connection with the accompanying figures and examples, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention. Also, as used in the specification including the appended claims, the singular forms "a," "an," and "the" include the plural, and reference to a particular numerical value includes at least that particular value, unless the context clearly dictates otherwise. When such a range is expressed, another embodiment includes from the one particular value and/or to the other particular value. Similarly, when values are expressed as approximations, by use of the antecedent "about," it will be understood that the particular value forms another embodiment. All ranges are inclusive and combinable.

As used herein, the term "antigen" ("Ag") refers to a substance that elicits an antibody response.

As used herein, the term "antigenic determinant" refers to part of an antigen molecule that binds to an antibody-combining site or to a T-cell receptor ("TCR").

, As used herein, the term "epitope" refers to an alternative term for an antigenic determinant. These are particular chemical groups on a molecule that are antigenic, *i.e*. that elicit a specific immune response.

As used herein, the term "hapten" refers to a compound, usually of low molecular weight, that is not itself immunogenic but, when bound to a carrier protein or cell, becomes immunogenic and induces antibodies, which can bind the hapten either alone or in the presence of a carrier.

As used herein, the term "expressible" refers to the ability of an antigen or molecule to be expressed by a cell.

As used herein, the term "carbohydrate antigens specifically expressible on a tumor cell surface" refers to the ability of tumor cells to express particular carbohydrate antigens in greater abundance compared to normal cells. Accordingly, the term "carbohydrate antigens specifically expressible on a tumor cell surface" is descriptive of the genus of carbohydrate antigens that are expressed an tumor cell surfaces in greater abundance compared to normal cells. Accordingly, the term "carbohydrate antigens specifically expressible on a tumor cell surface" is not meant to limit the disclosed inventions with regard to the origin of the carbohydrate antigens. Indeed, as is described further herein, the carbohydrate antigens can be provided by a variety of methods, including both naturally-derived sources as well as synthetic chemical pathways.

As used herein, the term "nanoparticle" refers to a particle of substance having a dimension, such as its diameter if the particle is spherical, smaller than about 100 nanometers ("nm").

As used herein, the term "conjugate" refers to a larger entity composed of two or more smaller entities that are linked together, connected together, associated together, or any combination thereof.

As used herein, the term "conjugating" refers to the process of linking, connecting, associating, or any combination thereof, two or more smaller entities to form a larger entity.

As used herein, the term "antigen-nanoparticle conjugate" refers to a conjugate of a nanoparticle and one or more antigens.

As used herein, the term "plurality" refers to a collection of two or more entities, the entities characterized as' being the same or different.

In various embodiments of the present invention, the antigen-nanoparticle conjugates include a plurality of carbohydrate antigens conjugated to one or more nanoparticles, wherein the antigens include TF antigen. A wide variety of carbohydrate antigens that are specifically expressible on a tumor cell surface are known can be conjugated to a wide variety of nanoparticles. Suitable carbohydrate antigens are typically displayed to the immune system in human carcinomas during tumor growth and progression. Suitable carbohydrate antigens can also be selected from carbohydrates that are prognostic indicators for cancer, markers of metastasized carcinoma cells, adhesion molecules involved in metastasis, as well as any combination thereof. Suitable carbohydrate antigens that are specifically expressible on a tumor cell surface are conveniently provided in a review by Hakomori, "Aberrant Glycosylation in Tumors and Tumor-Associated Carbohydrate Antigens", Adv. Cancer Res. 1989, 52, 257-331. This review provides two classification schemes of tumor-associated carbohydrate antigens, many of which are suitable in various embodiments of the present invention. These classification schemes are reproduced from Hakomori, pp. 262-263 in the following paragraph and table:

"Tumor-associated carbohydrate antigens can be divided into five classes according to their association with carrier molecules: (1) epitope structures clearly identified and expressed both in glycosphingolipids and glycoproteins; (2) those expressed only in glycosphingolipids; (3) those expressed only in glycoproteins; (4) polypeptide epitopes whose antigenicity is only maintained when a single or multiple threonine or serine residue(s) is glycosylated; (5) antigens whose epitope is poorly defined or undefined. The first class of antigens is characterized by lacto-series type 1 or type 2 chain (class 1; Table I,A). The second class of antigens are mainly globo- or ganglioseries glycosphingolipids (class 2; Table I,A). The third, fourth, and fifth classes are associated with glycoproteins, particularly mucin-type glycoproteins (classes 3-5; Table I,A). On the other hand, tumor-associated carbohydrate antigens can also be classified according to their specificity of expression, i.e., (1) antigens highly expressed in tumor cells, not expressed in the progenitor cells, but expressed in other types of normal cells and tissues (class 1; Table I,B); (2) those expressed highly in tumor cells but expressed weakly in their normal counterparts (class 2; Table I,B); and (3) those expressed exclusively in tumor cells, and not in normal cells (class 3; Table I,B). The majority of tumor-associated antigens belong to category (1) or (2), and only rarely to (3)."

| **TABLE 1** | |
|---|---|
| **Classification Of Tumor-Associated Carbohydrate Antigens** | |
| **A. Classification according to difference in epitope carrier** | |
| Class 1: **"A1"** | Associated with both glycolipid and glycoprotein (mostly lacto-series type 1 or type 2 chain) |
| Class 2: **"A2"** | Associated exclusively with glycolipid (mostly globo-series or ganglio-series structure) |
| Class 3: **"A3"** | Associated exclusively with glycoprotein (mostly related to mucin-type O-linked chains such as T, Tn, and Sialyl Tn) |
| Class 4: **"A4"** | Associated exclusively with glycoprotein whose structure is ill defined |
| Class 5: **"A5"** | Polypeptide epitopes whose antigenicity is influenced by O-glycosylation |
| | |

| **B. Classification according to specificity of antigen expression** | |
|---|---|
| Class 1: **"B1"** | Novel structures highly expressed in tumor cells, absent in their progenitor cells, but expressed in limited numbers in other normal cells. Examples: di- or trimeric Le^{x}, sialyl dimeric Le^{x} in various gastrointestinal and lung adenocarcinomas, and GD₂ and GD₃ gangliosides in melanoma and neuroblastoma |
| Class 2: **"B2"** | Common structures highly expressed in tumor cells, but present in lower quantity in various types of normal cells and tissues. Examples: GM₃ and GM₂ gangliosides in melanoma, and Gb₃ in Burkitt's lymphoma |
| Class 3: **"B3"** | Exclusively expressed in tumor cells, but absent (or present in immunologically undetectable levels) in normal cells. Examples: incompatible blood group antigens such as real A antigen and A-like antigen expressed in O tumors. The majority of A-like antigens are now identified as Tn antigen (see Class A3 above; see also Table V) |

Suitable additional carbohydrate antigens used in the present invention are typically selected from one or more of antigen Classes A2, A3, A4, A5, B2, and B3 of Table 1.

Preferred additional carbohydrate antigens include the saccharide portion of Tₙ-Antigen, Gb1 Antigen, GM1 Antigen, GM3 antigen, and other glycosphingolipid carbohydrates, LewisY tetrasaccharide, as well as their modifications, and any combination of these antigens. A More preferred carbohydrate antigen is Tn-Antigen. Although the antigen-nanoparticle conjugates are preferably prepared using exclusively one of these carbohydrate antigens, the antigen-nanoparticles of the present invention may suitably include at least one different carbohydrate antigen in addition to TF-Antigen. Accordingly, a plurality of different carbohydrate antigens can be conjugated to each nanoparticle in various embodiments of the present invention, and it is typically preferred that the plurality of carbohydrate antigens are identical to each other.

Suitable carbohydrate antigens used in the present invention typically are characterized as being composed of at least one disaccharide. In certain embodiments of the present invention, the at least one disaccharide typically includes at least one amino sugar group. Suitable amino sugar groups typically includes a six-member ring sugar having an amine group, examples of which include glucosamine and galactosamine.

Suitable carbohydrate antigens preferably are selected for their ability to induce or inhibit expression of a particular protein or carbohydrate in healthy cells. For example, in certain embodiments, carbohydrate antigens that are capable of inducing galectin-3 surface expression in endothelial cells are suitable used, especially in provide antigen-nanoparticle conjugates that aid in inhibiting adhesion of metastasizing cancer cells to healthy cells.

In various embodiments of the present invention it is desirable for at least one of the plurality of tumor-associated carbohydrate antigens to be conjugated to a spacer group, and the spacer group to be conjugated to the nanoparticle. Suitable spacer groups include a plurality of repeat groups, such as ethylene glycol, to form an oligomeric or polymeric chain. Each end of the oligomeric or polymeric chain is typically conjugated, respectively, to the nanoparticle and one of the carbohydrate antigens. Accordingly, suitable spacer groups include polyethyleneglycol chains, ("PEG"), *i.e*., -[OCH₂CH₂]ₙ, where n is 2 to about 100, as well as a carbon chain, a carbon chain including sulfur, nitrogen or oxygen in the backbone, polymers including polyacrylamide, a peptide chain made up of all glycine or alanine units, or any combination thereof. In certain embodiments including spacer groups, PEG with n in the range of about 4 to 16 is typically used.

In various embodiments of the present invention, the nanoparticles can be provided with linking groups. Accordingly, the antigen-nanoparticle conjugates may further include one or more carbohydrate antigens that are conjugated to one or more linking groups, wherein the linking groups are conjugated to the nanoparticle. Suitable linking groups include one or more sulfur atoms, carboxylate, amide, carbamate, carbonate, thiocarbamate, thiocarbonate, thioether, succinimide and n-hydroxy succinimide groups, or any combination thereof.

In certain embodiments, the antigen-nanoparticle conjugates include carbohydrate antigens that are each covalently linked to one or more sulfur atoms. Preferably, the antigen-nanoparticle conjugates of the present mvention include carbohydrate antigens that are each linked, individually, to one or more sulfur atoms. Carbohydrate antigens can be linked to one or more sulfur atoms as provided herein. Preferably, at least two sulfur atoms are covalently linked to each other. Certain antigen-nanoparticle conjugates of the present invention include at least one of the sulfur atoms is bonded to the nanoparticle. Suitable bonds between the sulfur atoms and the nanoparticle includes covalent bonds, and preferably includes van der Waals (*i.e*., dispersive bonds), or any combination thereof.

The antigen-nanoparticle conjugates of the present invention may also include one or more various other chemical groups, such as nucleic acids and amino acids. In these embodiments, the one or more various other chemical groups typically are linked to the nanoparticle, either through direct conjugation to the nanoparticle surface, or indirectly through use of a spacer, a linker, or any combination thereof. Typical embodiments can include carbohydrate antigens that are glycoproteins, *i.e.,* carbohydrate antigens conjugated to one or more amino acids.

The antigen-nanoparticle conjugates of the present invention may also include a combination of linking groups and spacer groups. Thus, certain embodiments of the present invention include one or more carbohydrate antigens that are conjugated to one or more spacer groups, the spacer groups are conjugated to one or more linking groups, and the one or more linking groups are conjugated to the nanoparticle. Although any of the aforementioned linking groups are suitable in these embodiments, it is preferred that the linking groups include one or more sulfur atoms.

Suitable nanoparticles used in the present invention are composed of a plurality of atoms that are capable of forming a particle having a dimension of about 100 nm or smaller. A wide variety of nanoparticles are known that can be used in the present invention, although suitable nanoparticles of the present invention may be composed of any combination of atoms in the periodic table, atoms are typically selected from one of more of Groups 1 to 17, more typically from one or more of Groups 3 to 16, even more typically from Groups 4 to 15, and even more typically from Groups 5 to 14. Preferred nanoparticles are composed of metal or semiconducting elements, such as those found in Groups 8 to 14, and of these, metal atoms of Groups 10 to 12 being more preferred, with copper, gold, silver, platinum, rhodium and palladium being the most preferred atoms, as well as any combination thereof.

A variety of nanoparticles can be prepared using known techniques or purchased from commercial sources. For example, U.S. Patent No. 5,879,715 describes processes and systems for production of inorganic nanoparticles, U.S. Patent No. 6,361,944 describes the preparation of gold nanoparticles having oligonucleotides attached thereto, U.S. Patent No. 6,530,944 describes a variety of optically-active nanoparticles, for example gold nanoparticles and nanoshells, for use in therapeutic and diagnostic methods, U.S. Patent No. 6,602,932 describes nanoparticle composites and nanocapsules for guest encapsulation and methods for synthesizing same, and U.S. Patent No. 6,780,301 methods of manufacture of colloidal rod particles as nanobar codes. Various colloidal compositions also include nanoparticles that are suitable for use in the present invention. U.S. Patent No. 4,853,335 describes that colloidal gold particles can be prepared in a number of ways through the reduction of chloroauric acid which produces a variety of particle sizes ranging from 5 nm to 100 nm. This patent also describes that colloidal gold particles can have an intermediary binder (*i.e.* linker) absorbed to its surface.

Suitable nanoparticles used in the present invention each typically have from about 50 to about 10,000 atoms, more typically from about 100 to about 1000 atoms; and even more typically from about 200 to about 800 atoms. Likewise, suitable nanoparticles have a dimension typically in the range of from about 0.5 nm to about 200 nm, even more typically in the range of from about 1 nm to about 100 nm, and even further typically in the range of from about 1 nm to about 10 nm. The nanoparticles that are composed of a collection of 50 to 10,000 atoms bonded in a variety of ways can give rise to variety of shapes. Accordingly, suitable nanoparticles of the present invention can include any one or more of the following types of shapes: icosahedral, rod-like, cubic, rhombic, hexagonal, fullerene, and typically spheroid, as well as filled, hollow, layered and shell-like versions of these shapes, as well as any combinations thereof.

. In various embodiments of the present invention, the number of carbohydrate antigens conjugated to each of the nanoparticles is typically in the range of from about 2 to about 1000, more typically in the range of from about 10 to 500, and even more typically in the range of from about 30 to 200. Although a plurality of antigen-nanoparticle conjugates can be produced wherein each has the same number of conjugated carbohydrate antigens, typically the antigen-nanoparticle conjugates have a distribution of number of conjugated carbohydrate antigens. Accordingly, a plurality of the antigen-nanoparticle conjugates is suitably characterized is having a distribution of conjugated antigens that can be further expressed in statistical terms know to those skilled in the art, for example, the mean number of conjugated antigens. Likewise, the plurality of nanoparticles will also be suitably characterized in terms of one or more distributions, for examples, particle size, number of atoms, atomic composition, or any combination thereof. Accordingly, suitable antigen-nanoparticle conjugates of the present invention will typically have a molecular weight in the range of from about 1,000 Daltons to about 1 million Daltons, more typically in the range of from about 10,000 Daltons to about 500,000 Daltons, and even more typically in the range of from about 25,000 Daltons to about 150,000 Daltons. Accordingly, a plurality of antigen-nanoparticle conjugates will typically be characterized as having a distribution of molecular weights, such as a weight-average molecular weight or in terms of a number-average molecular weight.

In various embodiments of the present invention for preparing antigen-nanoparticle conjugates, the methods typically include providing at least one nanoparticle and conjugating a plurality of carbohydrate antigens to the nanoparticle, wherein the carbohydrate antigens are specifically expressible on a tumor cell surface. Suitable nanoparticles and suitable carbohydrate antigens are described above. During conjugation, a plurality of nanoparticles are provided in a suitable reaction zone, and a plurality of carbohydrate antigens are typically conjugated to at least a portion of the nanoparticles within the reaction zone. Suitable reaction zones include a vessel, chamber or conduit suitable for conducting chemical reactions. Although it is typical for a portion of the carbohydrate antigens to remain unconjugated to the nanoparticles in the reaction zone, it is preferred that substantially all of the plurality of carbohydrate antigens are conjugated to each of the nanoparticles.

In other embodiments of the method of preparing antigen-nanoparticle conjugates, prior to conjugating the carbohydrate antigens to the nanoparticles, the carbohydrate antigens may be conjugated to a linking group. Examples of linking groups are provide above, with specific examples provided below.

In other embodiments of the method of preparing antigen-nanoparticle conjugates, the nanoparticles can be linked with one or more linking groups. In this regarding, linking groups can be provided on the surface of the nanoparticles through by reacting any one or more of the above-mentioned linking groups with a plurality of nanoparticles in a reaction zone.

In certain preferred embodiments of the present invention, the method of conjugating the carbohydrate antigens to the nanoparticle uses a self-assembly procedure. Self-assembly methods are described in U.S. Patent Application Pub. No. 2004/0018633A1, which discloses thiol terminated monodisperse ethylene oxide oligomer capped gold nanoclusters (*i.e*., nanoparticles). Preferably, the carbohydrate antigens terminated with one or more linking groups sulfur atoms are self-assembled onto at least one surface of the nanoparticle.

In certain embodiments of the methods of the present invention, linking groups are conjugated to the nanoparticle, and at least one of the carbohydrate antigens is conjugated to at least one of the nanoparticle linking groups. In addition, various embodiments of the present invention also include conjugating one or more spacer groups to the carbohydrate antigens and to the nanoparticle. Any of the variety of spacer groups provided herein can be used in these methods. Preferably, as provided in further detail in the examples below, the spacer groups include PEG.

Additional process steps may also be included in the methods of the present invention. In particular, it is desirable in certain embodiments, to purify the resulting antigen-nanoparticle conjugates. Suitable methods of purifying the nanoparticles include filtration, centrifugation, electrophoresis, chromatography, crystallization, or any combination thereof.

In other aspects of the present disclosure there are provided methods for inhibiting metastasis of carcinoma cells in a mammal. These methods typically include administering to a mammal or cells thereof a therapeutically effective amount of at least one of the antigen-nanoparticle conjugates described herein. Accordingly, in one aspect of the present disclosure there are provided methods for inhibiting metastasis of carcinoma cells in a mammal, that include administering to a mammal or cells thereof a therapeutically effective amount of antigen-nanoparticle conjugates, the nanoparticle conjugates comprising a plurality of carbohydrate antigens conjugated to a plurality of nanoparticles, wherein the carbohydrate antigens are specifically expressible on a tumor cell surface. Based on the *in vivo* mouse studies provided herein, one skilled in the art can readily determine suitable dosage regimens for preventing the metastasis in other mammals, such as humans. Accordingly, one aspect of the present disclosure involves administering to a human or cells thereof a therapeutically effective amount of antigen-nanoparticle conjugates, the nanoparticle conjugates comprising a plurality of carbohydrate antigens conjugated to a plurality of nanoparticles, wherein the carbohydrate antigens are specifically expressible on a tumor cell surface. A human that is diagnosed as having cancer, for example, breast cancer, can be treated using the antigen-nanoparticle conjugates and methods of the present invention. In certain aspect the treatment of humans using the various methods and antigen-nanoparticle conjugates gives rise to inhibition of metastasis, especially to the lungs. During treatment of a mammal, certain aspect of the present disclosure may further include the steps of removing primary tumor cells from the mammal. Typically, the tumor cells are removed from the mammal prior to administering the therapeutically effective amount of the antigen-nanoparticle conjugates. In this regard, primary tumor cells that are inhibited from adhering to, and colonizing on, secondary tissues of the mammal.

In various aspect of the present disclosure for treating a mammal, suitable antigen-nanoparticle conjugates include any of the antigen-nanoparticle conjugates described herein. For example, suitable antigen-nanoparticle conjugates include carbohydrate antigens that are displayed to the immune system in human carcinomas during tumor growth and progression. Other antigen-nanoparticle conjugates that are particularly useful use carbohydrate antigens that include TF-Antigen, Tₙ-Antigen, Gb1 Antigen, GM1 Antigen, GM3 antigen, and other glycosphingolipid carbohydrates, LewisY tetrasaccharide, as well as their modifications, and any combination of these antigens. More preferred carbohydrate antigens are selected from TF-Antigen and Tn-Antigen, or any combination thereof.

A nanoparticle is conjugated when the nanoparticle has carbohydrate antigen groups linked to the surface of the nanoparticle which can act to change the response of a biological system from that resulting from contact with a non-conjugated nanoparticle. The term "link" refers to an attractive association of an atom or molecule with another atom or molecule, for example, a covalent bond, an ionic bond, a hydrogen bond, or a bond or interaction of another type. As an example, carbohydrate antigen groups may be attached to the surface of the nanoparticle to allow the nanoparticle as a whole to adhere to biological material, and render the nanoparticle as a whole biologically inert so that the biological system does not "see" the nanoparticle and does not respond to the nanoparticle. A biological material can include a secretion, *e.g*., an antibody, from another biological material.

In the context of interactions with biological entities, such as cells, or chemical constituents thereof, *e.g.,* receptors, the term `binding" is used consistently with usage in the biological literature. That is, "binding" can refer to the net attractive force between two molecules or macromolecular structures resulting from a number of different, simultaneously acting types of bonds including ionic bonds, hydrogen bonds, and van der Waals interactions.

The carbohydrate antigen group can be linked to the nanoparticle, or it can be linked to a linker or spacer group that is conjugated to the nanoparticle. Certain saccharides are carbohydrate antigen groups. In this application, the term "saccharide" refers to mono-, di-, tri-, and oligosaccharides. The saccharide can be a saccharide found in nature, or can be a saccharide which is not found in nature. A saccharide may be, for example, an antigen found on the membrane of a tumor cell or a bacterium. For example, Thomsen-Friedenreich disaccharide is found on the surface of many human cancer cells but not on the surface of normal human cells. A saccharide found on the surface of cancer cells, but not on the surface of normal human cells can be referred to as a tumor-associated carbohydrate antigen.

The term "linked" is used herein to mean either directly linked or indirectly linked. In a first example, a first chemical group is directly linked to a second chemical group if there is a link between an atom or a portion of the first chemical group, and a link between an atom or a portion of the second chemical group. In a second example, a first chemical group is indirectly linked to a second chemical group if there is a link between an atom or a portion of the first chemical group and a third chemical group, and another link between the third chemical group and a second chemical group. Referring to a first chemical group as "linked" to a second chemical group means that the link could be direct, as in the first example, or indirect, as in the second example.

A chemical group, *e.g*., a carbohydrate antigen group, can be linked to the nanoparticle directly, or it can be linked to the nanoparticle indirectly, for example, through a linking group. A linking group can play a number of different roles. For example, a linking group may act as a "spacer" between the nanoparticle and the carbohydrate antigen group so that the carbohydrate antigen group can assume a conformation required to stimulate or suppress the response of a biological system as desired. A linking group can also act to separate charge in or on the nanoparticle from the carbohydrate antigen group. A linking group can facilitate linking a carbohydrate antigen group to the nanoparticle. For example, a carbohydrate antigen group can be linked to a linking group, the linking group can include an atom which has a high affinity for the nanoparticle and thus links to the nanoparticle or integrates with the nanoparticle. For example, a carbohydrate antigen group can be linked to a sulfur atom, the sulfur atom serving as a linking group, and the sulfur atom can in turn be linked to the surface of the nanoparticle. As another example, a saccharide which is a carbohydrate antigen group can be linked to a linking group including a chain of at least one carbon atom. The linking group can further include a sulfur atom. The sulfur atom can then be linked to the nanoparticle, for example, a gold or silver nanoparticle. An atom which links directly to the nanoparticle, for example, a sulfur atom, can be referred to as part of a linking group or as a linking group in itself. Alternatively, an atom which links directly to the nanoparticle can be referred to as outside of a linking group. That is, the previous example could also be presented as a carbohydrate antigen group linked to a linking group including a chain of at least one carbon atom, with the linking group linked to the sulfur atom, which is linked to the nanoparticle. In an embodiment, a Thomsen-Friedenreich disaccharide is covalently bonded to a linking group including a chain of five carbon atoms, which is in turn linked to a sulfur atom, which is in turn linked to the nanoparticle of silver or gold.

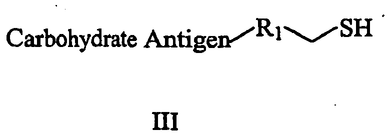

In an embodiment, the carbohydrate antigen group-thiol of Formula III can be formed by reacting a glycoside of Formula I with a alkylthio acid in the presence of a catalyst to produce a thioester of Formula II, in which R₂ represents a group containing one or more carbon atoms. The thioester of Formula II can then be debenzylidinated and hydrolyzed to produce the carbohydrate antigen group-thiol of Formula III in solution. In an embodiment, the glycoside can be selected to produce a Thomsen-Friedenreich-thiol for the compound of Formula III.

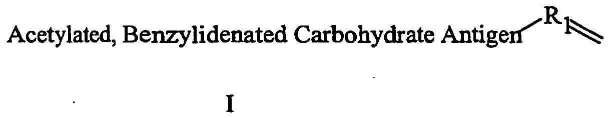

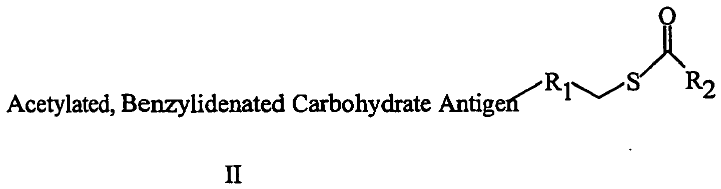

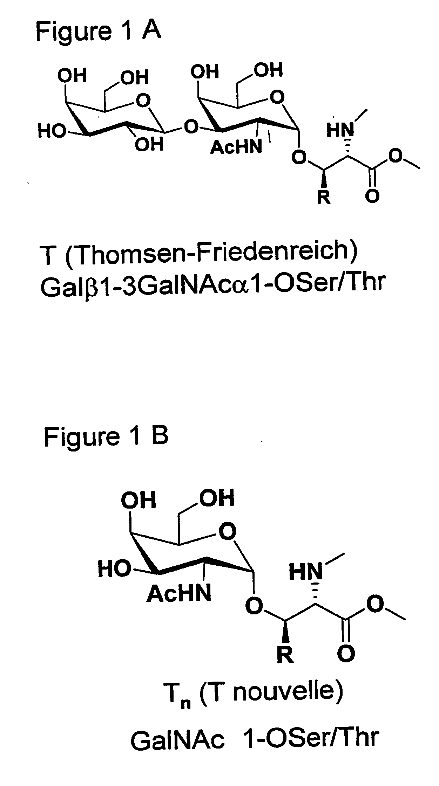

In an embodiment, the solution containing conjugated nanoparticles illustrated in Fig. 1 can be purified, and the purified solution can be dried to isolate a preparation of carbohydrate antigen group-conjugated nanoparticles. For example, the solution can be filtered through a membrane with a cutoff in the range of 10,000 to 1 million daltons. The cutoff can be selected so that only the desired nanoparticles less than a certain size pass through and larger nanoparticles and particles are retained; in this case the permeate passing through the filter is dried to obtain isolated conjugated nanoparticles. Alternatively, the cutoff can be selected so that the desired nanoparticles of greater than a certain size are retained and smaller nanoparticles and particles pass through; in this case the retentate retained by the filter is dried to isolate conjugated nanoparticles. The solution containing the nanoparticles can also be forced through a filter with a larger cutoff, the permeate then passed through a filter with a smaller cutoff, and the retentate of the filter with the smaller cutoff then dried to isolate conjugated nanoparticles. Membranes of various types can be used, for example, an ultrafiltration membrane can be used or a dialysis membrane can be used. As an example, the solution containing the nanoparticles can be passed through an ultrafiltration membrane with a cutoff of about 50 kilodaltons and the retentate dried to isolate conjugated nanoparticles. The isolated conjugated nanoparticles can be redissolved or resuspended in an aqueous solvent, for example, a biocompatible aqueous solvent, for further use. A biocompatible aqueous solvent could be a solvent containing components in addition to water and the nanoparticles which improve the performance of the water-dissolved or water-suspended nanoparticles when they are applied to a biomaterial. For example, a biocompatible aqueous solvent may be adjusted to have similar salinity and pH as a tissue into which it is to be injected.

In an embodiment, a conjugated nanoparticle is linked to a cell to form a cell-nanoparticle complex. For example, the carbohydrate antigen group on the nanoparticle may act as a ligand which couples with a receptor on the surface of a cell. The carbohydrate antigen group on the nanoparticle can be, for example, a saccharide, such as Thomsen-Friedenreich disaccharide. For example, the Thomsen-Friedenreich disaccharide may act as a ligand which couples with a receptor protein, galectin-3, on an endothelial cell. In addition to a carbohydrate antigen group, the nanoparticle may have other groups on the surface of the nanoparticle , such as a mercaptoalkanoic acid, *e.g*., mercaptoacetic acid.

In an embodiment, the conjugated nanoparticles are in the form of a formulation. Such a formulation includes a liquid and conjugated nanoparticles dissolved or suspended in the liquid so that the solution or suspension does not precipitate or flocculate. The conjugated nanoparticles according to the invention, when mixed with water, form a solution which is clear, although it may be colored. Thus it appears that the nanoparticles dissolve in water. However, the literature on hydrophilic nanoparticles often refers to a suspension of nanoparticles, it may be that although when mixed with water, the resultant composition is clear, the term "suspension" is used because of the greater size of nanoparticles with respect to low molecular weight molecules.

Carbohydrate antigen-conjugated nanoparticles according to the invention can be injected into an organism, for example, into the tissues, including the circulatory system, of a living animal. For example, the carbohydrate antigen-conjugated nanoparticles can be dissolved or suspended in a biocompatible aqueous solvent, and the solution or suspension then injected into the body. The Thomsen-Friedenreich-conjugated nanoparticles of the invention would adhere to cells which express galectin-3, in particular, endothelial cells which have been - stimulated to express large amounts of galectin-3. Adhesion of tumor cells on to the endothelial cells would then be inhibited based upon a masking effect The fact that the carbohydrate antigen-conjugated nanoparticles of the present application are water-soluble and biocompatible makes them particularly advantageous for use in evaluating tissue in vivo or in vitro.

Nanoparticles can also be conjugated with biological receptors which couple with antigens on cancer cells, these antigens either not being present in normal cells or being present on cancer cells in much greater concentration than in normal cells. Similarly, nanoparticles can be conjugated with antigens which couple with receptors on cancer cells, these receptors either not being present in normal cells or being present on cancer cells in much greater concentration than in normal cells. By contacting the nanoparticle conjugates with tissue in the body or in an in vitro sample, adhesion by cancer cells can be inhibited.

Carbohydrate antigen-conjugated nanoparticles can be used in therapeutic applications. For example, cancer cells may express antigens which couple with receptors on normal cells. Such coupling can play a role in metastasis of cancer cells or other interactions of cancer cells with the body. In an embodiment, nanoparticles are functionalized with the same antigens which the cancer cells express, the nanoparticles may bind to receptors on normal cells and thereby block adhesion of cancer cells to the normal cells. For example, as discussed above, cancer-associated carbohydrate T antigen, *e.g.*, Thomsen-Friedenreich disaccharide, plays a leading role in docking breast and prostate cancer cells onto endothelium by specifically interacting with an endothelium-expressed protein, galectin-3. Thomsen-Friedenreich-functionalized nanoparticles are injected into the body to adhere to endothelial cells which express galectin-3, in particular, endothelial cells which have been stimulated to express large amounts of galectin-3, and thereby block adhesion of the cancer cells to the endothelium. Such therapy could delay or prevent the metastasis of cancer cells. See Glinsky et al., "The role of Thomsen-Friedenreich antigen in adhesion of human breast and prostate cancer cells to the endothelium", Cancer Res., 61 (12): 4851-4857, June 15, 2001.

Without being bound by any particular theory of operation, multiple presentations of antigenic saccharides to receptor proteins, *i.e.*, a high concentration of antigenic saccharides, may dramatically increase the strength of coupling between the particle or cell with the antigenic saccharides and the particle or cell with the receptor proteins; this is known as the cluster glycoside effect. Thus, carbohydrate antigen-conjugated nanoparticles can advantageously be used as vehicles to provide antigenic saccharides to receptors proteins, because the antigenic saccharides are present in high concentrations on the nanoparticles.

The carbohydrate antigen-conjugated nanoparticles presented in this application can be especially useful in that they can be used for therapy and diagnosis. For example, carbohydrate antigen-conjugated nanoparticles can be injected into the body for therapy, and then induced to fluoresce and imaged to monitor the response of the body, especially of diseased tissue, to the therapy.

As discussed above, nanoparticles functionalized with an antigen can bind with diseased cells, *e.g.*, cancer cells, which express a receptor for the antigen, and nanoparticles functionalized with a receptor can bind with diseased cells, *e.g*., cancer cells, which express an antigen which couples with the receptor. In an embodiment, the nanoparticle, in addition to the carbohydrate antigen, has an additional therapeutic agent linked to it. By injecting such a nanoparticle, site-specific drug delivery can be achieved. Such site-specific therapeutic agent delivery is of great interest in cancer therapy, as the therapeutic agents used can be toxic to normal as well as cancerous cells. The therapeutic agent delivered can be a drug, *e.g.*, a drug to stimulate an immune response, a chemotherapeutic agent, *e.g*., for killing or weakening a cancer cell, or a radiotherapeutic agent for killing or weakening a cancer cell. Alternatively, the nanoparticle portion of the antigen-nanoparticle conjugate may itself serve as the therapeutic agent. For example, radioisotopes may be used as elements in the formation of the nanoparticle. Non-radioactive elements or compounds may be selected for their toxicity to cancer cells and selected so that the nanoparticle degrades over time, exposing the cancer cells to which the antigen-nanoparticle conjugate is bound to these toxic elements or compounds. Drug-functionalized, radioactive, or chemotoxic nanoparticles functionalized with an antigen can also be used to selectively weaken or destroy cells in the body which cancer cells co-opt for their growth or proliferation.

In an embodiment, carbohydrate antigen-conjugated nanoparticles are used as a component of an immunogenic composition. Tumor-associated antigens expressed by cancerous cells, for example, antigenic saccharides such as Thomsen-Friedenreich disaccharide, can be used to functionalize nanoparticles. Introduction of tumor-associated-antigens alone usually fails to stimulate an immune response because of immune self-tolerance. However, without being bound by a particular theory of operation, multiple and dense presentation of tumor-associated-antigens on the surface of a nanoparticle core may be recognized by the immune system as distinctly unnatural so that an immune response is stimulated. When injected into the body, these tumor-associated antigen-functionalized nanoparticles stimulate an immune response and thus spur the immune system in attacking the cancerous cells.

### EXAMPLES

In these examples we report an efficient and facile preparation of Tn-antigen (also referred to herein as "Tn Antigen" and "Tn Ag", Tₙ antigen, and similar variants) and TF-antigen (also referred to as "TF Ag", "TF antigen", "T Antigen, and similar variants") encapsulated gold nanoparticle conjugates, their interaction with galactoside-specific protein galectin-3 and TF-antigen binding 15-mer peptide P-30. Applications in imaging, early cancer diagnostics, and their use as antimetastatic agents are discussed.

### Results and Discussion

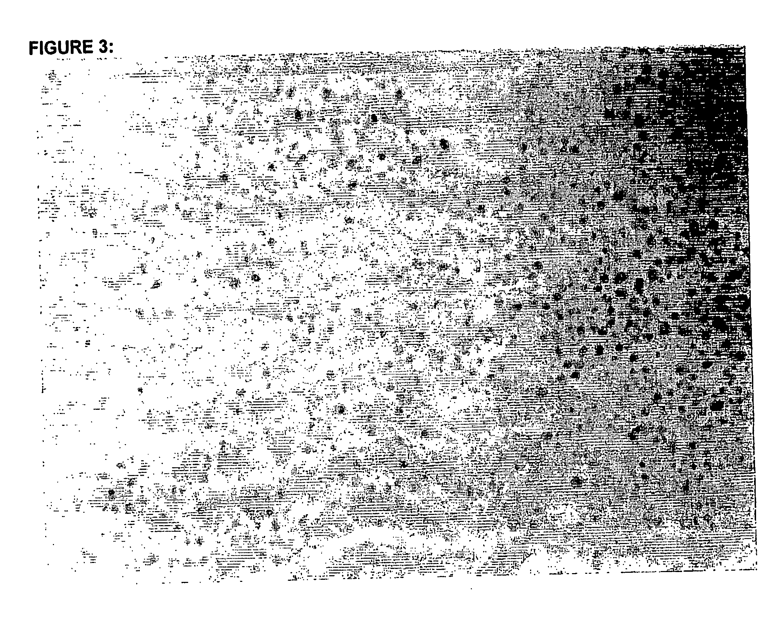

The preparation of disulfide-bridged Tₙ- and TF antigens is outlined in **Scheme** 1 of Svarovsky, S. A.; Barchi, J. J. Jr. Carbohydr. Res. 2003, 338, 1925-1935. We then prepared Tₙ- and TF-antigen-encapsulated gold nanoparticle conjugates by a modified procedure of Brust et al. ("Synthesis of Thiol-derivatized Gold Nanoparticles in a Two-phase Liquid-Liquid System", J. Chem. Soc., Chem. Commun. 1994, 801-802.) The resulting antigen-nanoparticle conjugates were extremely soluble in water but completely insoluble in methanol and other organic solvents. The TEM measurements (**Figure 3**) shows that solubilization in water did not result in any aggregation of the nanoparticles..

The disappearance of the methylene signals next to the -SH group in the TF-antigen conjugated nanoparticles ("TF-AuNP") as well as the disappearance of -SH peak from the IR spectrum are clearly indicative that the gold particle was bound with the TFSH monolayer. The chemical shifts and the integrals of the rest of protons confirm that the bonded TFSH molecules have the same structure as the free ones. Results of elemental analysis of the gold nanoparticles showed same relative C/H/N/O ratio as in the free ligand.

### EXPERIMENTAL

### Materials and Methods

Melting points were determined on Fisher-Johns melting point apparatus and are uncorrected. R_{f} values refer to TLC performed on Analtech Uniplates GF pre-coated with silica gel 60 to a thickness of 0.25 mm. The spots were visualized by charring with a solution of ammonium molybdate (IV) tetrahydrate (12.5 g) and cerium (IV) sulfate tetrahydrate (5.0 g) in 10% aqueous H₂SO₄ (500 mL). Flash column chromatography was performed under medium pressure using silica gel 60 (230-400 mesh, E. Merck) and usually employed a stepwise solvent polarity gradient, correlated with TLC mobility.

NMR spectra were recorded on Varian InovaUnity-400 instrument with residual CHCl₃ (7.26 ppm) as the internal standard at frequencies of 399.74 MHz for ¹H and 100.51 MHz for ¹³C. Assignments were based on gCOSY, TOCSY, ROESY, and ¹³C/DEPT experiments. ¹H NMR data are tabulated in the order of multiplicity (s, singlet; d, doublet; dd, doublet of doublets; dt, double of triplets; t, triplet; q, quartet; m, multiplet; brs, broad signal), number of protons, and coupling constant(s) in hertz. IR spectra were taken with JASCO FT/IR-615 spectrometer. Specific optical rotations were determined using JASCO-P1010 polarimeter in 0.5 dm cuvette at 589 nm in chloroform. Five consecutive measurements were taken each time; the average value is given. Positive ion fast-atom bombardment mass spectra (FABMS) were obtained at an accelerating voltage of 6 kV and a resolution of 2000. Glycerol was used as the sample matrix, and ionization was effected by xenon atoms. Laser scanning confocal microscopy was performed on Zeiss 510 confocal microscope (NCI-Frederick, Confocal Microscopy Facility, Image Analysis Lab). Elemental analyses were performed by Atlantic Microlab, Inc., Norcross, GA. Unless otherwise noted, materials were purchased from Aldrich-Sigma (Milwaukee, WI) and used without further purification.

### Thioacetoxypentyl-2-deoxy-4,6-benzylidene-2-acetamido-α-D-galactopyranoside (126C-287)

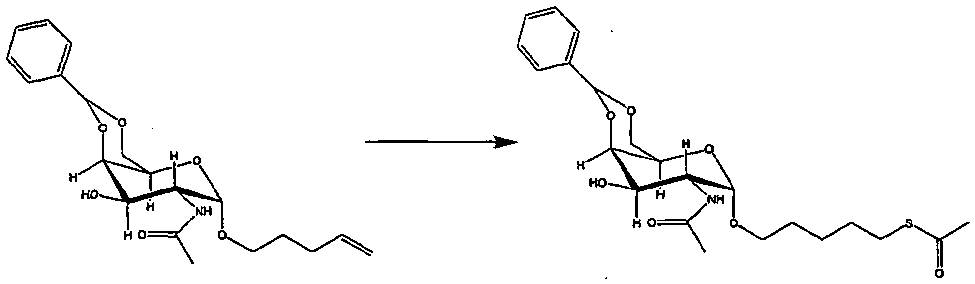

To a solution of 200 mg (0.53 mmols) of **126C-284** in 6 ml of anhydrous 1,4-dioxane was added 4.4 ml (8.0 mmols, 15.08 equivs) of freshly distilled (3x) thiolacetic acid followed by addition of 90 mg (0.09 mmols) of AIBN. Reaction was left stirring at 75°C. In 6 hours TLC (5% v/v MeOH in CH₂Cl₂) showed no starting material. Reaction was co-evaporated with xylenes to remove the solvents and the residual oil was flash-chromatographed with 20:1 EtOAc/hexanes to give 208 mg (0.46 mmols, 86% yield) of white solid. R_{f} 0.22, (10:1 EtOAc/Hexanes); [α]_{D} = +210.00 (c 0.21 in CHCl₃); IR (neat) 3291.89, 2114.56, 1748.16, 1649.80, 1554.34, 1375.00, 1218.79; ¹H NMR (400 MHz, CDCl₃) δ = 7.49-7.56 (m, 2H, Ph), 7.35-7.42 (m, 3H, Ph), 5.76 (d, 1H, J=9.37Hz, NH), 5.58 (s, 1H, PhCH), 4.94 (d, 1H, J=3.51, H'-1), 4.46 (m, 1H, H'-2), 4.27 (dd, 1H, J=1.17, 12.49Hz, H'-6b), 4.23 (d, 1H, J=3.51Hz, H'-4), 4.08 (dd, 1H, J=1.95, 12.49Hz, H'-6a), 3.84 (ddd, 1H, J=3.5, 10.5, 14.4Hz H'-3), 3.70 (dt, 1H, J=6.25, 9.76Hz, -OCH₂CH₂CH₂CH₂CH₂SAc), 3.67 (bs, 1H, H'-5), 3.44 (dt, 1H, J=6.25, 9.76Hz -OCH₂CH₂CH₂CH₂CH₂SAc), 2.88 (m, 2H, -OCH₂CH₂CH₂CH₂CH₂SAc), 2.76 (d, 1H, J=10.93Hz, -OH), 2.34 (s, 3H, SAc), 2.05 (s, 3H, NHAc), 1.56-1.68 (m, 4H, - OCH₂CH₂CH₂CH₂CH₂SA_{C}), 1.38-1.49 (m, 2H, -OCH₂CH₂CH₂CH₂CH₂SAc); ¹³C NMR (100 MHz, CDCl₃) δ = 171.37, 129.36, 128.43, 126.56, 101.51, 98.38, 75.71, 69.58, 69.35, 68.04, 63.15, 50.60, 30.86, 29.55, 29.04, 28.98, 25.55.
FAB MS m/z: 454.2 (MH⁺). Anal: Calcd. for C₂₂H₃₁NO₇S: C 58.26; H 6.89; N 3.09. Found: C 58.14; H 6.80; N3.02.

### Thioacetoxypentyl-2-deoxy-2-acetamido-α-D-galactopyranoside (126C-289-2)

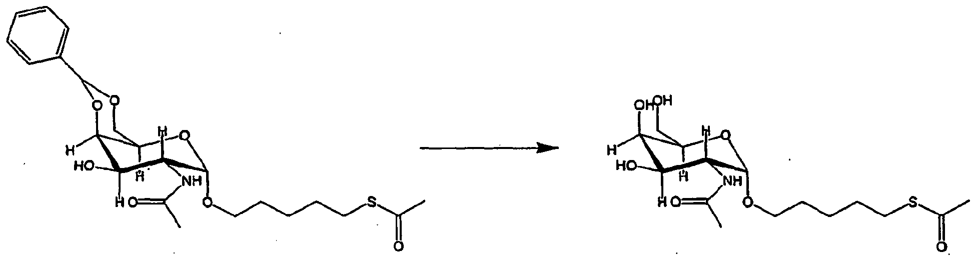

A solution of 180 mg (0.40 mmols) of **126C-287** in 10 ml of 80% AcOH was allowed to react at 60 °C for 3 hours. No starting material could be detected after this time. Reaction solution was diluted with xylenes and rotoevaporated to dryness. The white solid residue was chromatographed on silica gel using 10% v/v MeOH in CH₂Cl₂ as eluent. This gave 80 mg (56% yield) of the α-product. R_{f} 0.21, (10% MeOH in CH₂Cl₂); [α]_{D} = +104.74 (c 0.18 in CHCl₃); 1R (neat) 3319.86, 2119.40, 1738.80, 1692.23, 1643.05, 1542.77; ¹H NMR (400 MHz, CDCl₃) δ = 6.00 (d, 1H, J=8.20, NH); 4.86 (d, 1H, J=3.9Hz, H'-1) 4.32 (m, 1H, H'-2), 4.02 (d, 1H, J=3.12Hz, H'-4), 3.86 (bd, 1H, OH), 3.76 (m, 1H, H'-3), 3.71 (dt, 1H, J=6.64, 9.76Hz, - OCH₂CH₂CH₂CH₂CH₂SAc), 3.49 (bs, 1H, OH), 3.43 (dt, 1H, J=6.25, 9.76Hz - OCf₂CH₂CH₂CH₂CH₂SAc), 2.81-2.87 (m, 2H, -OCH₂CH₂CH₂CH₂CH₂SAc), 2.67 (bs, 1H, OH), 2.34 (s, 3H, SAc), 2.09 (s, 3H, NHAc), 1.57-1.69 (m, 4H, -OCH₂CH₂CH₂CH₂CH₂SAc), 1.38-1.48 (m, 2H, -OCH₂CH₂CH₂CH₂CH₂SAc); ¹³C NMR (100 MHz, CD₃OD) δ = 196.46, 172.68, 97.51, 71.23, 69.18, 68.49, 67.47, 61.62, 50.48, 48.44, 47.17, 29.40, 29.32, 28.71, 28.58, 25.26, 21.50.
FAB MS m/z: 366.2 (MH⁺). Anal: Calcd. for C₁₅H₂₇NO₇S: C 49.30; H 7.45; N 3.83. Found: C 49.38; H 7.34; N 3.83

### Thiopentyl-2-deoxy-2-acetamido-α-D-galactopyranoside (126C-290)

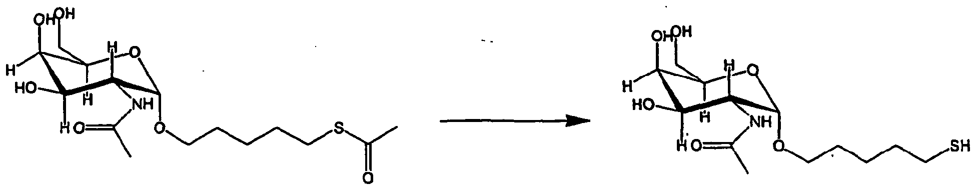

To a solution of 60 mg (0.16 mmols) of **126C-289-2** in 3 ml of anhydrous MeOH. 45 µl (10.6 mg, 0.20 mmols) of NaOMe (25% w/v solution in MeOH) was added dropwise at 0 °C. The solution was then warmed to ambient temperature and stirred for an additional hour. The solution was neutralized with weakly acidic ion-exchange resin (Amberlite^{®}-50). The resin was filtered and washed with 20 ml of MeOH. FCC on silica gel using 10% v/v MeOH in CH₂Cl₂ afforded 47 mg (89%) of white solid. R_{f} 0.10, (10% MeOH in CH₂Cl₂); ¹H NMR (400 MHz, D₂O) δ = 4.76 (d, 1H, J=3.51, H'-1), 3.99 (dd, 1H, J=3.90, 10.93Hz, H'-2), 3.84 (d, 1H, J=3.51Hz, H'-6a), 3.80 (d, 1H, J=5.86Hz, H'-4), 3.77 (dd, 1H, J=3.12, 3.51H, H'-3), 3.62 (d, 1H, J=1.95Hz, H'-6b), 3.60 (bs, 1H, H'-5), 3.53-3.60 (m, 1H, - OCH₂CH₂CH₂CH₂CH₂SNa), 3.30-3.38 (m, 1H, -OCH₂CH₂CH₂CH₂CH₂SNa), 2.42 (t, 1H, J=7.03, 7.42Hz, -OCH₂CH₂CH₂CH₂CH₂SNa), 1.90 (s, 3H, NHAc), 1.42-1.53 (m, 4H, - OCH₂CH₂CH₂CH₂CH₂SNa), 1.26-1.36 (m, 2H, -OCH₂CH₂CH₂CH₂CH₂SNa), (check H4,5,6 assignments, blow up COSY); ¹³C NMR (100 MHz, D₂O) δ = 174.69, 96.96, 71.00, 68.69, 68.07, 67.84, 61.42, 50.23, 32.78, 28.04, 24.25, 23.86, 22.15. FAB MS m/z: 346.2 (M+Na⁺). Anal: Calcd. for C₁₃H₂₄NNO₆S: C 45.21; H 7.00; N 4.06. Found: C 46.31; H 7.80; N 4.15

### Tn-Encapsulated Gold Nanoparticles (Tn-AuNP)

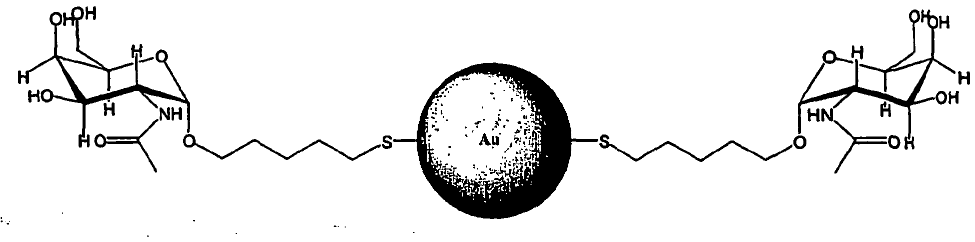

A solution of HAuCl₄ (1 ml, l5mmol/L, 0.015 mmols) in water was combined with 1 ml of 35 mmol/L (0.035 mmols) solution of tetraoctylammonium bromide (0.035 mmols) in toluene. The two-layered solution was vigorously stirred until the water layer became clear (complete transfer of HAuCl₄ into the organic phase). The orange toluene layer was separated and transferred into a reaction vial. Solution of 3.5 mg (0.0054 mmols) of **126C-300** and 1 mg of NaBH₄ in 2 ml MeOH was added. The combined solution was then slowly added to the toluene solution of HAuCl₄ with vigorous stirring. Immediate precipitation of dark insoluble material occurred. After addition was complete, the reaction solution was stirred for additional hour. The precipitate was centrifuged at 5,000 rpm for 15 min and washed with MeOH. This procedure was repeated three times. The dark precipitate was dissolved in water and lyophilized. NMR of the nanoparticles in D₂O showed traces of starting material (NT=5,000) along with broad signals corresponding to the sugar ligand **126C-300.** The nanoparticles were additionally purified by centrifugal filtering on Centricon^{®}-3 until no starting material could be detected by NMR and TLC. ¹H NMR spectrum (400 MHz, D2O) of Tn-AuNP showed two broad peaks at 1.00-2.50 ppm and 3.00-4.5 ppm. The chemical shifts of the peaks correspond to those of compound **126C-300.** Previous reports indicate that the broadened peaks may be attributed to the assembly of thiolated Tn-antigen on the gold surface.(Lin, C-C. et al JACS, 2002, 124, 3508 and refs therein) IR, NMR, TEM, UV-Vis.

### Preparation of TF-AuNP

### 126B-172

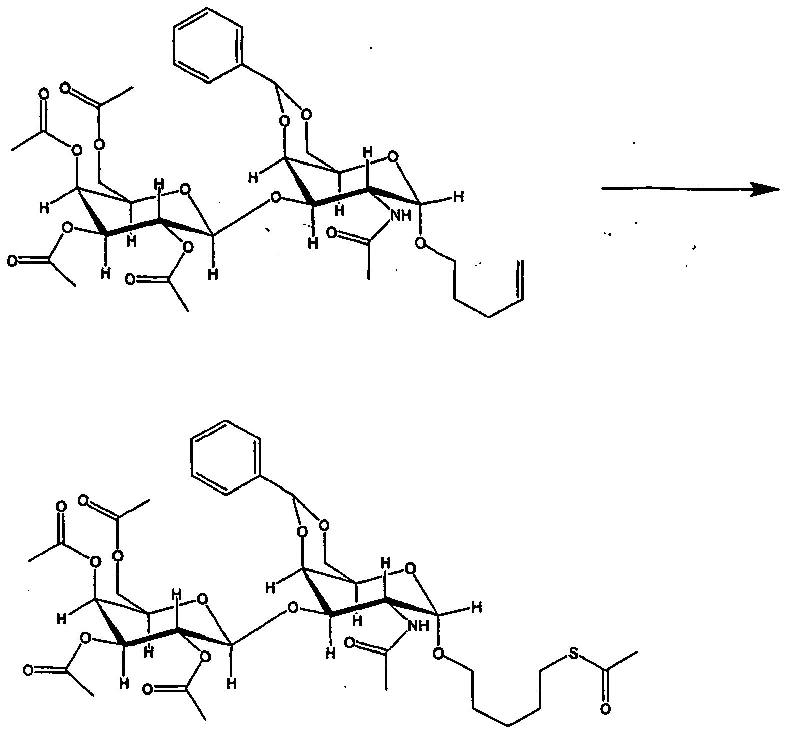

A solution of 120 mg (0.17 mmols) of **126B-164**{Svarovsky & Barchi 2003 281 /id} in 4 ml of anhydrous 1,4-dioxane was purged with argon for 20 min. To this deoxygenated solution 1.4 ml (2.55 mmols, 15 eqiuvs) of triply distilled thiolacetic acid was added followed by 30 mg (0.03 mmols) of AIBN. The reaction was left to stir under argon atmosphere at 75°C until no starting material could be detected by TLC (approximately 1 hour). The reaction was then quenched with cyclohexene (0.1 ml). The solution was co-evaporated with xylenes under reduced pressure. FCC on silica gel with 3:1 EtOAc/ hexanes provided 125 mg (99.8%) of white solid (7:3 mixture of amide rotamers). R_{f} 0.19 (3:1 EtOAc/hexanes); [α]_{D} = +157.79 (c 0.22 in CHCl₃); IR (neat) 3099.05, 1752.01, 1689.34, 1368.25, 1220.72; ¹H NMR (400 MHz, CDCl₃) δ = 7.48-7.54 (m, 2H, Ph), 7.27-7.38 (m, 3H, Ph), 5.56 (d, 1H, J=8.98Hz, NH), 5.52 (s, 1H, PhCH), 5.34 (d, 1H, J=2.34Hz, H"-4), 5.13-5.19 (m, 1H, H"-2), 4.92-4.98 (m, 2H, H"-3, H'-1), 4.76 (d, 1H, J=7.81Hz, (βH"-1), 4.62 (m, 1H, H'-2), 3.90-4.30 (m, 6H, H'-6, H"-6, H'-4, H"-5), 3.88 (m, 1H, H'-3), 3.59 (bs, 1H, H'-5), 3.60-3.70 (m, 1H, -OCH₂CH₂CH₂CH₂CH₂SAc), 3.35-3.45 (m, 1H, -OCH₂CH₂CH₂CH₂CH₂SAc), 2.83 (t, 2H, J=7.42Hz, -OCH₂CH₂CH₂CH₂CH₂SAc), 2.30 (s, 3H, SAc), 1.93, 1.96, 2.00, 2.01, 2.11 (s, 15H, OAc, NHAc), 1.52-1.62 (m, 4H, - OCH₂CH₂CH₂CH₂CH₂SAc), 1.34-1.44 (m, 2H, -OCH₂CH₂CH₂CH₂CH₂SA_{C}),¹³C NMR (100
MHz, CD₃Cl) δ = (need ¹³C). FAB MS m/z: 784.0 (MH⁺). Anal: Calcd. for C₃₆H₄₉NO₁₆S: C 55.16; H 6.30; N 1.79. Found: C 54.89; H 6.32; N 1.87

### 126B-175

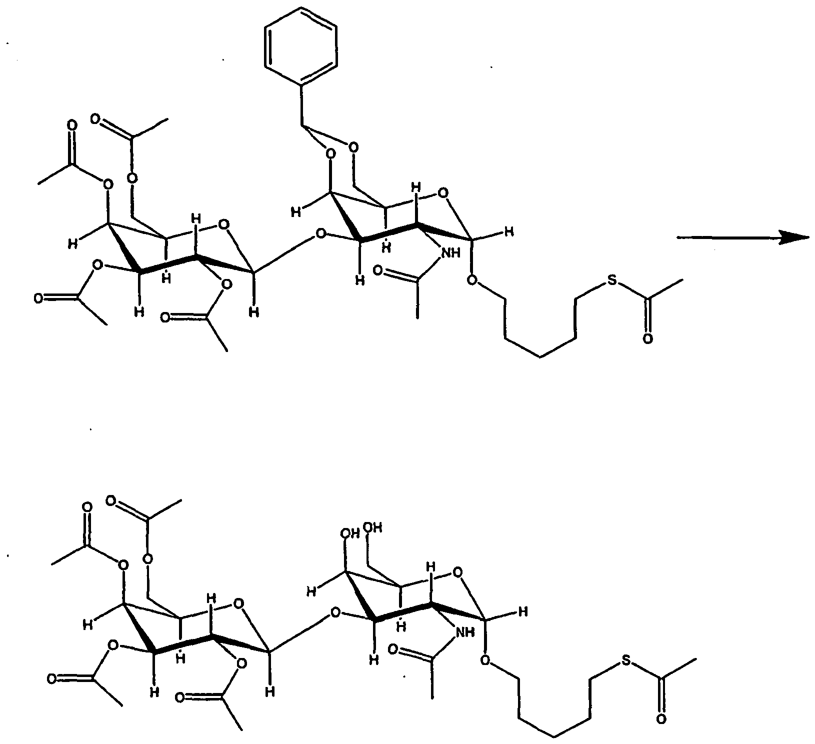

A solution of 110 mg (0.14 mmols) of **126B-172** in 3 ml of 80% AcOH was stirred at 45°C for 16 hours. The reaction solution was concentrated at reduced pressure and co-evaporated twice with xylenes. The residue was flash chromatographed on silica gel using 7% MeOH in CH₂Cl₂ to provide 69 mg (71% yield) of white foam (7:3 mixture of amide rotamers). MP 95-97 °C; R_{f} 0.46 (10% MeOH in CH₂C1₂); [α]_{D} = +107.69 (c 0.15 in CHCl₃); IR. (neat); 3552.24, 1750.08, 1688.37, 1656.55, 1545.67, 1370.18, 1220.72; ¹H NMR (400 MHz, CDCl₃) δ = 5.33 (dd, 1H, J=0.78, 3.51Hz, H"-4), 5.11-5.18 (m, 1H, H"-2), 4.94 (dd, 1H, J=3.51, 10.54Hz, H"-3), 4.78 (d, 1H, J=3.51Hz, H'-1), 4.61 (d, 1H, J=8.20Hz, (βH"-1), 4.51 (td, 1H, J=3.90, 10.54Hz, H'-2), 3.65-4.18 (m, 8H, H'-5, H'-3, H'-6, H"-6, H'-4, H"-5), 3.60-3.67 (m, 1H, - OCH₂CH₂CH₂CH₂CH₂SAc), 3.31-3.40 (m, 1H, -OCH₂CH₂CH₂CH₂CH₂SAc), 2.78-2.85 (m, 2H, -OCH₂CH₂CH₂CH₂CH₂SAc), 2.70 (bs, 2H, OH), 2.28 (s, 3H, SAc), 1.92, 1.94,2.00, 2.02, 2.11 (s, 15H, OAc, NHAc), 1.50-1.62 (m, 4H, -OCH₂CH₂CH₂CH₂CH₂SAc), 1.32-1.42 (m, 2H, - OCH₂CH₂CH₂CH₂CH₂SAc), ¹³C NMR (100 MHz, CDCl₃) δ = 196.46, 170.61, 170.39, 170.32, 169.72, 169.55, 101.86, 97.87, 78.38, 71.07, 70.82, 69.69, 69.37, 68.83, 67.89, 67.10, 62.88, 61.52, 48.06, 30.86, 29.57, 29.03, 28.88, 25.61, 23.56, 20.87, 20.81, 20.79, 20.72, 20.67.

FAB MS m/z: 696.1 (MH⁺). Anal: Calcd. for C₂₉H₄₅NO₁₆S: C 50.06; H 6.52; N 2.01. Found: C 49.55; H 6.53; N 2.00

### 126B-177L

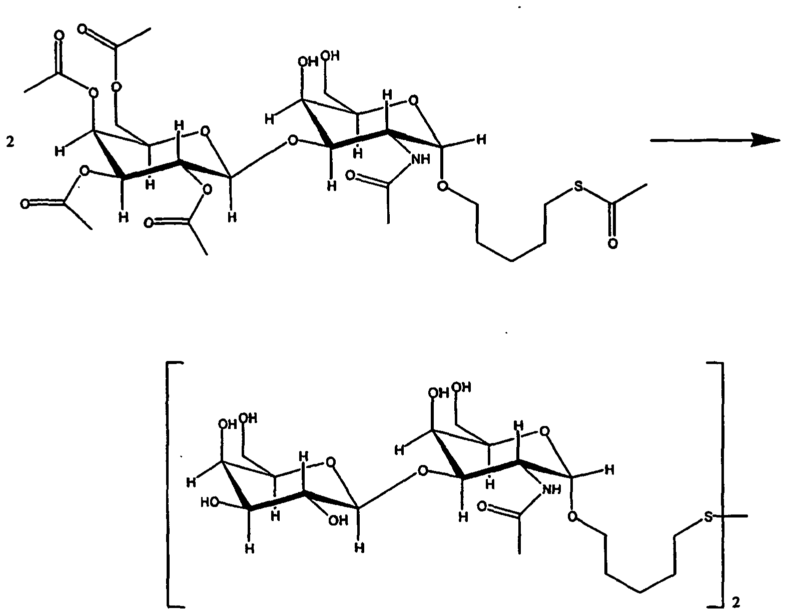

A solution of 300 mg (0.43 mmols) of **126B-175** in 5 ml of MeOH was treated with 30 µl of 25% (w/v) NaOMe/MeOH. The reaction was stirred at RT and with for 24 hours with slow flow of air bubbling through the solution. Intitially, two spots were observed by TLC (R_{f} 0.13 and R_{f} 0.00 in 20% v/v MeOH/CH₂Cl₂). The higher Rf spot corresponds to the monomeric thiol 126B-177H while the lower spot corresponded to the desired dimer **126B-177L.** After 24 hours only one spot of 126B-177L was observable. At this point, the reaction was carefully neutralized with strongly acidic Amberlite^{®}-120, and evaporated under reduced pressure at 50°C to give 200 mg (96%) of NMR pure **126B-177L.** The crude product was purified by RPFC with 10% → 40% (v/v) MeOH/H₂O to give 187 mg (90%) of pure **126B-177L** as a white powder. The compound was both soluble in water and methanol. MP 247-249 °C; [α]_{D} = +93.76 (c 1.6 in MeOH); IR (neat) 3379.64, 2944.77, 2827.13, 2112.64, 1746.23, 1218.79; ¹H NMR (400 MHz, CD₃OD) δ = 4.14 (d, 2H, J= 1.95Hz, NH), 3.87 (dd, 2H, J= 3.12, 10.93Hz, H'-2), 3.68 (m, 2H, [-OCH₂-]₂), 3.40 (m, 2H, -OCH₂-), 2.68 (t, 4H, J= 7.42Hz, -CH₂S-SCH₂-), 1.94 (s, 6H, NHAc), 1.43-1.74 (m, 12H, [-OCH₂CH₂CH₂CH₂CH₂S-]₂); ¹³C NMR (100 MHz, CDCl₃) δ = 172.77 (NHAc), 105.08 (C-1), 97.60, 77.76, 75.52, 73.52, 71.33, 70.85, 69.07, 68.83, 67.54, 61.64, 61.38, 49.19, 38.41, 28.89, 28.81, 24.93, 21.69. FAB MS m/z: 969.2 (MH⁺). Anal: Calcd. for C₃₈H₆₈N₂O₂₂S(3xH₂O) C, 44.61; H, 7.29; N, 2.74. Found: C 44.24; H 7.21; N 2.73.

### Synthesis of HEXPEG linkers

### 126E-170

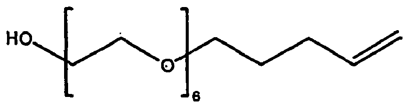

### {PALEGROSDEMANGE, SIMON, et al. 1991 171 /id}

A solution of 533µl of 50% NaOH (6.7 mmols) was added to 10g (35.5 mmols) of hexa(polyethyleneglycol) (Aldrich) at 100 °C. The reaction was stirred for 30 min at 100 °C. 5-Bromo-1-pentene (1g, 6.7 mmols) was quickly added and the reaction was stirred at 100 °C for 24 hours. The reaction was diluted with water and extracted 6x with EtOAc. Combined organic extracts were evaporated and separated by FCC with 10:1 CH₂Cl₂/MeOH. This gave 1 g of the title product as a clear liquid. R_{f} (10:1 CH₂Cl₂/MeOH) = 0.3; IR (neat); 3465 (brs), 2231, 2022, 1690; ¹H NMR (400 MHz, CD₃Cl) δ = 5.73-5.83 (m, 1H, CH=CH₂), 4.90-5.02 (m, 2H, CH=CH₂), 3.53-3.70 (m, 24H, PEG), 3.43 (t, 2H, J= 6.49Hz, OCH₂CH₂CH₂CH=CH₂), 2.08 (m, 2H, OCH₂CH₂CH₂CH=CH₂), 1.65 (m, 2H, OCH₂CH₂CH₂CH=CH₂); ¹³C NMR (100 MHz, CD₃Cl) δ = 72.49, 70.66, 70.59, 70.55, 70.52, 70.33, 70.07, 61.68, 30.19, 28.75. FAB MS m/z: 351.3 (MH⁺), 373.3 (M+Na⁺). Anal: Calcd. for C₁₇H₃₄O₇: C, 58.26; H, 9.78; Found: C 58.30; H 9.78.

### 126E-179

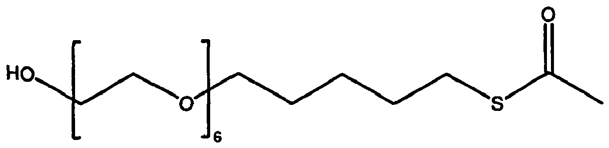

Palegrosdemange C, et al., J. Am. Chem. Soc., 113(1):12-20 Jan 2, 1991

A solution of 2.8 g (8mmols) of **126E-170** in 30 ml MeOH was treated with 30 mmols) (2.3g, 2.3 ml) of freshly distilled HSAc. Solution was purged with argon for 20 minutes and 10mg of AIBN was added. Reaction was irradiated with 360nm light overnight, quenched with 1 ml of cyclohexene and evaporated. The residue was purified by FCC with 15:1 CH₂Cl₂:MeOH to afford 3g (88%) of S-acetylated product as a clear liquid. IR (neat); 3465 (brs), 1689; ¹H NMR (400 MHz, CD₃Cl) δ = 3.52-3.72 . (m, 24H, PEG), 3.42 (t, 2H, J= 6.49Hz, OCH₂CH₂CH₂CH₂CH₂SAc), 2.84 (d, 2H, J= 6.95Hz, OCH₂CH₂CH₂CH₂CH₂SAc), 2.60 (brs, 1H, OH), 2.29 (s, 3H, SAc), 1.56 (m, 4H, OCH₂CH₂CH₂CH₂CH₂SAc), 1.39 (m, 2H, OCH₂CH₂CH₂CH₂CH₂SAc); ¹³C NMR (100 MHz, CD₃Cl) δ = 195.91, 72.47, 71.09, 70.61, 70.57, 70.55, 70.36, 70.09, 61.72, 30.62,29.34,29.10,29.01,25.36; FAB MS m/z: 427.4 (MH⁺). Anal: Calcd. for C₁₉H₃₈O₈S: C, 53.50; H, 8.98; 0,30.01. Found: C 52.17; H 8.91.

### 126F-018

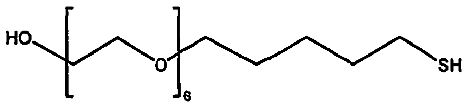

A solution of 1g (2.34 mmols) of **126E-179** in 15 ml of dry MeOH was treated with 10 drops of 25% w/v NaOMe/MeOH for 30 minutes. Reaction was quenched with Amberlite^{®}-120 ion-exchange resin, filtered and concentrated. The residue was purified by fcc with 15:1 CH₂Cl₂:MeOH to afford 350mg (40%) of disulfide which was cleaved to free thiol with DTT as follows. The solution of 350 mg of the disulfide was treated with excess 10x DTT in H₂O for 1 hour, concentrated and purified by FCC with 10% CH₂C1₂:MeOH to give 340 mg (98%) of **126F-018** as pale yellow liquid. ¹H NMR (400 MHz, CD₃Cl) δ = 3.48-3.68 (m, 24H, PEG), 3.38 (t, 2H, J = 6.49 Hz, OCH₂CH₂CH₂CH₂CH₂SH); 2.80 (t, 1H, J = 6.03 Hz, OH), 2.46 (m, 2H, OCH₂CH₂CH₂CH₂CH₂SH), 1.54 (m, 4H, OCH₂CH₂CH₂CH₂CH₂SH), 1.38 (m, 2H, OCH₂CH₂CH₂CH₂CH₂SH), 1.28 (m, 1H, SH); ¹³C NMR (100 MHz, CD₃Cl) δ = 72.49, 71.08, 70.51, 70.48, 70.29, 70.05, 61.61, 33.75, 29.01, 24.87, 24.46. FAB MS m/z; 385.3 (MH⁺), 407.3 (M+Na⁺). Anal: Calcd. for C₁₇H₃₆O₇S: C, 53.10; H, 9.44; Found: C 52.53; H 9.41.

### Synthesis of β-Linked Galactoside

### 126F-083

A solution of 1.4 g (2.8 mmols) of **Gal-TCI {Ren** & Liu 1999 124 /id} and 1 g (2.34 mmols) of **126E-179** in 20 ml of anhydrous CH₂Cl₂ was transferred via canula to 500 mg of flame-dried MS (4A) and stirred at RT for 30 min prior to addition of 20 µl of TMSOTF. After 1 hour at RT the reaction was quemched with 0.1 ml Et₃N, filtered through Celite^{®}, concentrated and purified by fcc on silica gel yielding 1.2 g (82%) of **126F-083** as a glassy solid. ¹H NMR (400 MHz, CDCl₃) δ = 5.31 (m, 1H, H-4), 5.14 (dd, 1H, J = 7.94 Hz, J = 10.48 Hz, H-3); 4.95 (dd, 1H, J = 3.44 Hz, J = 10.46 Hz, H-2), 4.50 (d, 1H, J = 7.99 Hz, H-1), 3.45-3.72 (m, 24H, 24H PEG6), 3.80-4.20 (m, 3H, H-5, H-6ab), 3.40 (t, 2H, J = 6.60 Hz, - OCH₂CH₂CH₂CH₂CH₂SAc), 2.70 (t, 2H, J = 7.32 Hz, -OCH₂CH₂CH₂CH₂CH₂SAc), 2.25 (s, 3H, SAc), 2.08, 1.99, 1.98, 1.91 (s, 12H, 4xAc), 1.52 (m, 4H, -OCH₂CH₂CH₂CH₂CH₂SAc), 1.35 (m, 2H, -OCH₂CH₂CH₂CH₂CH₂SAc); ¹³C NMR (100 MHz, CDCl₃) δ = 170.36, 170.25, 170.14, 169.45, 101.38, 71.11, 70.91, 70.59, 70.27, 70.10, 69.07, 68.81, 67.07, 61.29, 30.62, 29.36, 29.11, 29.02, 25.38, 20.76, 20.67, 20.58; FAB MS m/z: 795.6 (M + K⁺); Anal: Calcd. for C₃₃H₅₆O₁₇S: C, 52.37; H, 7.46; Found: C 52.09; H 7.49.

### 126F-084

A solution of 1.2 g (1.92 mmols) of **126F-083** in 15 ml MeOH was treated with catalytic amount of 25% NaOMe/MeOH. After stirring overnight under argon the reaction was neutralized with strongly acidic Amberlite^{®}-120 ion-exchange resin, filtered, evaporated and purified by rp-fcc on C-18 column with 10% → 40% of MeOH in H₂O to give after lyophilization 750 mg (94%) of **126F-084** as a glassy solid. ¹H NMR (400 MHz, D₂O) δ = 4.30 (d, 1H, J = 7.80 Hz, H-1), 3.97 (dt, 1H, J = 4.07 Hz, J = 11.19 Hz, ), 3.80 (m, 1H, ), 3.50-3.77 (m, 29H, 24H PEG6, 5H sugar ring), 3.44 (m, 3H, -OCH₂CH₂CH₂CH₂CH₂SH, H-2), 2.45 (t, 2H, J = 7.1 Hz, -OCH₂CH₂CH₂CH₂CH₂SH), 1.50 (m, 4H, -OCH₂CH₂CH₂CH₂CH₂SH), 1.32 (m, 2H, -OCH₂CH₂CH₂CH₂CH₂SH); ¹H NMR (400 MHz, CDCl₃) δ = 4.25 (d, 1H, J = 7.56 Hz, (βH-1) 4.07 (brs, 4H, OH), 3.97 (m, 1H, H-4), 3.78 (m, 1H), 3.50-3.73 (m, 28H, PEG6), 3.42 (t, 2H, J = 6.54 Hz, -OCH₂CH₂CH₂CH₂CH₂SH), 2.48 (m, 2H, -OCH₂CH₂CH₂CH₂CH₂SH), 1.57 (m, 4H, - OCH₂CH₂CH₂CH₂CH₂SH), 1.40 (m, 2H, -OCH₂CH₂CH₂CH₂CH₂SH); ¹³C NMR (100 MHz, CDCl₃) δ = 103.57, 74.47, 73.60, 71.12, 70.52, 70.48, 70.45, 70.39, 70.26, 70.05, 68.77, 68.54, 61.38, 33.77, 29.02, 24.88, 24.49; FAB MS m/z: 585.5 (M + K⁺); Anal: Calcd. for C₂₃H₄₆O₁₂S: C, 50.53; H, 8.48; Found: C 48.99; H 8.51.

### Synthesis of β-Linked Lactoside

### 126F-093

A solution of 0.96 g (1.23 mmols) of O-lactosyl trichloroacetimidate heptaacetate and 426 mg (1 mmol) of PEG6-Pent-SAc in 15 ml of anhydrous CH₂Cl₂ was added via canula to 500 mg of flame-dried MS (4A). After stirring for 30 min at RT the solution was treated with 15 µl of TMSOTF. The reaction was quenched in 1 hour by addition of few drops of Et₃N, filtered through Celite^{®} and evaporated. The residue was purified by fcc to give 1.05 g (82%) of the product as a glassy solid. ¹H NMR (400 MHz, CDCl₃) δ = 5.35 (m, 1H, H-4'), 5.11 (dd, 1H, J_{1,2} = 7.89 Hz, J_{2,3} = 10.42 Hz, H-2"), 4.95 (dd, 1H, J_{3,4} = 3.44 Hz, J_{2,3} = 10.44 Hz, H-3"), 4.89 (dd, 1H, J = 7.95 Hz, J = 9.56 Hz, H-4'), 4.55 (d, 1H, J = 7.95 Hz, H-1"), 4.78 (d, 1H, J = 7.85 Hz, H-1'), 4.05-4.17 (m, 3H, H-6"ab, H-2'), 3.75-3.95 (m, 3H, H-6'ab, H-5), 3.50-3.75 (m, 24H, PEG6), 3.45 (t, 2H, J = 6.60 Hz, -OCH₂CH₂CH₂CH₂CH₂SAc), 2.87 (t, 2H, J = 7.30 Hz, -CH₂SAc), 2.32 (s, 3H, SAc), 2.15, 2.12, 2.06, 2.05, 2.04, 2.03, 1.97 (s, 21H, Ac), 1.59 (m, 4H, - OCH₂CH₂CH₂CH₂CH₂SAc), 1.40 (m, 2H, -OCH₂CH₂CH₂CH₂CH₂SAc); ¹³C NMR (100 MHz, CDCl₃) δ = 170.34, 170.14, 170.04, 169.74, 169.65, 169.05, 101.12, 100.69, 71.12, 70.68, 70.58, 70.10, 66.52, 62.02, 60.77, 29.36, 29.12, 29.03, 25.38, 20.69, 20.56, 20.44. FAB MS m/z: 1045.3 (MH⁺), 1083.4 (M + K⁺). Anal: Calcd. for C₄₅H₇₂O₂₅S: C, 51.72; H, 6.94, Found: C 51.34; H 6.91.

### 126F-094

A solution of 860 mg (0.82 mmols) of **126F-093** in 20 ml MeOH was treated with catalytic amount of 25% w/v NaOMe in MeOH. In 1 hour reaction was quenched with strongly acidic ion-exchange resin Amberlite^{®}-120, filtered, concentrated and purified by rp-fcc on C-18 column with 20% → 40% MeOH in H₂O. ¹H NMR (100 MHz, D₂O) δ = 4.40 (d, 1H, J = 7.92 Hz, (βH-1"), 4.34 (d, 1H, J = 7.72 Hz, βH-1'), 3.20-4.00 (m, 38H, 24 PEG6, 2 Pent, 12 sugar), 2.45 (t, 2H, J = 7.01 Hz, CH₂SH), 1.51 (m, 4H, -OCH₂CH₂CH₂CH₂CH₂SH), 1.33 (m, 2H, -OCH₂CH₂CH₂CH₂CH₂SH); ¹³C NMR (100 MHz, D₂O) δ = 102.96, 102.91, 102.10, 78.30, 75.32, 74.73, 74.26, 72.77, 72.51, 70.88, 69.57, 69.11, 68.69, 68.53, 68.48, 60.97, 60.06, 32.73, 27.97, 24.01, 23.65. FAB MS m/z: 709.4 (MH⁺), 747.3 (M + K⁺). Anal: Calcd. for C₂₉H₅₆O₁₇S: C, 49.14; H, 7.96, Found: C 48.75; H 7.77.

### Synthesis of α- and β-HEXPEG-Linked Thomsen-Friedenreich Antigen

### 126E-181-HI

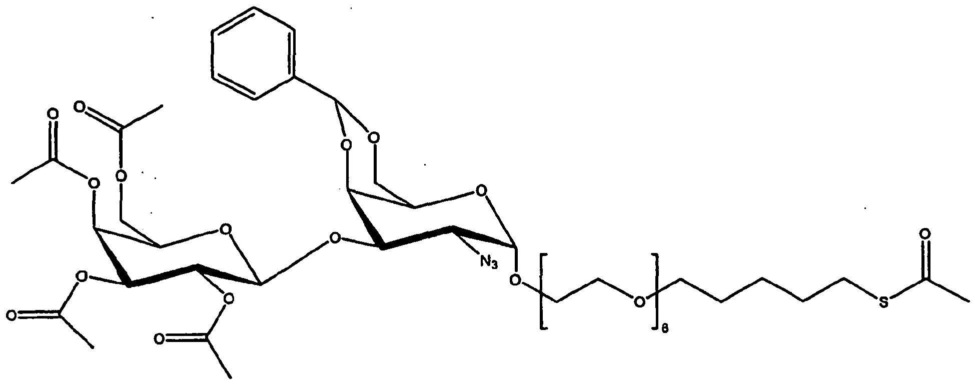

A solution of 2.5g (3.26 mmols) of **126C-219** and 1g (2.36 mmols) of **126E-179** in CH₂Cl₂ was evaporated and co-evaporated with toluene and the residue was dried under vacuum overnight. The dried mixture was dissolved in 3:1 mixture of anhydrous CH₂Cl₂/THF (60:20ml) and added via canula to 2g of flame-dried MS (4Å). After stirring for 20 minutes 15µl TMSOTF was quickly injected via syringe. After 30 min reaction was quenched with Et₃N, filtered and evaporated. Residue was purified by fcc with 20:1 CH₂Cl₂:MeOH to afford 1.5g of α-anomer and 400 mg of β-anomer (α: β=3.75). Total yield 79%. Other reaction conditions have been tested to achieve higher α/β stereoselectivity. It has been noted that lowering temperature leads to lower α/β selectivity. Increasing polarity resulted in better α/β selectivity. Using 3:1 mixture of CH₂Cl₂:THF at ambient temperature gave the highest stereoselectivity.

Desired a-anomer **(126E-181-HI)** was isolated as a glassy solid. R_{f} (10:1 CH₂Cl₂:MeOH)= 0.8; 4:1 ratio of rotamers. NMR of the major rotamer is given. ¹H NMR (400 MHz, CD₃Cl) δ = 7.39-7.43 (m, 2H, Ph), 7.20-7.28 (m, 3H, Ph), 5.43 (s, 1H, PhCH), 5.28 (m, 1H, H-4"), 5.17 (dd, 1H, J = 7.88, 10.20 Hz, H-2"), 4.98 (d, 1H, J = 3.71 Hz, H-1'), 4.92 (m, 1H, H-3"), 4.67 (d, 1H, J = 7.88 Hz, H-1"), 4.26 (m, 1H, H-4'), 3.8-4.15 (m, 8H, H-2', H-3', H-6', H-6", H-5', H-5"), 3.52 (m, 24H, PEG), 3.32 (m, 2H, OCH₂CH₂CH₂CH₂CH₂SA_{C}), 2.74 (m, 2H, OCH₂CH₂CH₂CH₂CH₂SAc), 2.19 (s, 3H, SAc), 2.04, 1.94, 1.92, 1.86 (s, 12H, 4xAc), 1.46 (m, 4H, OCH₂CH₂CH₂CH₂CH₂SAc), 1.30 (m, 2H, OCH₂CH₂CH₂CH₂CH₂SAc); ¹³C NMR (100 MHz, CD₃Cl) δ= 195.86, 170.28, 170.23, 170.11, 169.38, 137.73, 128.81, 128.22, 128.08, 126.50, 126.13, 102.41, 100.58, 98.87, 75.81, 71.06, 70.77, 70.55, 70.50, 70.22, 70.07, 69.19, 68.67, 67.51, 66.97, 63.02, 62.39, 61.37, 58.84, 30.61, 29.64, 29.34, 29.09, 28.99, 25.35, 20.69, 20.67, 20.54; FAB MS m/z: 1031.9 (MH⁺), 1069.9 (M+K⁺). Anal: Calcd. for C₄₆H₆₉N₃O₂₁S: C, 53.53; H, 6.74; N, 4.07; O, 32.55; S, 3.11 Found: C xx.xx; H x.xx.

Side-product **126E-181-LO** was isolated as a glassy solid. R_{f} (10:1 CH₂Cl₂:MeOH)= 0.5;

### 126E-188-HI

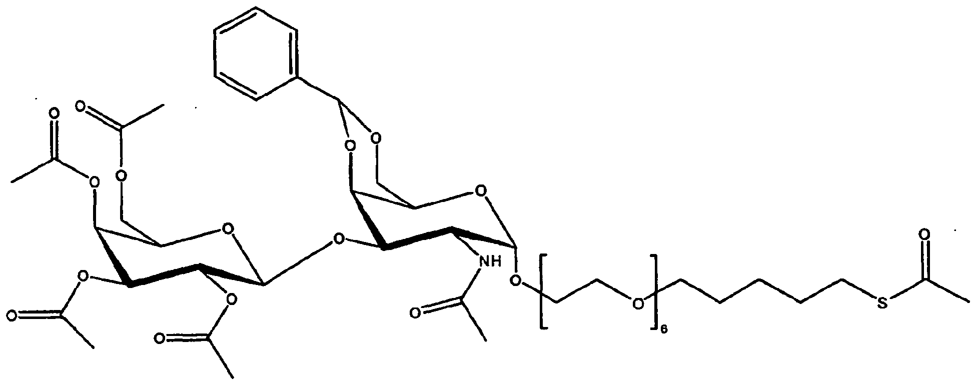

A solution of 1.5g of **126E-181-HI** in 30ml THF + 15ml AcOH + 5ml Ac₂O was treated with 15g of Zn dust. In 4 hours reaction was filtered through a pad of Celite^{®}, the zinc cake was washed with EtOAc. Filtrate was washed successively with water, sat. NaHCO₃ and brine. Organic layer was dried over Mg₂SO₄, evaporated and flash-chromatographed with 20:1 CH₂Cl₂:MeOH to afford 1.2g (79%) of 126E-188-HI as a glassy solid. R_{f} (20:1 CH₂Cl₂:MeOH) = 0.2; [α]_{D} = +72.38 (c 1.37 in CHC1₃); ¹H NMR (400 MHz, CD₃Cl) δ = 7.52-7.57 (m, 2H, Ph), 7.30-7.40 (m, 3H, Ph), 5.96 (d, 1H, J = 9.27 Hz, NH), 5.56 (s, 1H, PhCH), 5.38 (m, 1H, H-4"), 5.20 (dd, 1H, J = 7.88, 10.66 Hz, H-2"), 4.99 (m, 2H, H-1', H-3"), 4.77 (d, 1H, J = 7.88 Hz, H-1"), 4.67 (m, 1H, H-2'), 3.72-4.30 (m, 8H, H-2', H-3', H-6', H-6", H-5', H-5"), 3.65 (m, 24H, PEG), 3.44 (t, 2H, J = 6.49 Hz, OCH₂CH₂CH₂CH₂CH₂SAc), 2.86 (t, 2H, J = 7.42 Hz, OCH₂CH₂CH₂CH₂CH₂SAc), 2.32 (s, 3H, NHAc), 2.15 (s, 3H, SAc), 2.05, 2.04, 1.98, 1.97 (s, 12H, 4xAc), 1.59 (m, 4H, OCH₂CH₂CH₂CH₂CH₂SAc), 1.42 (m, 2H, OCH₂CH₂CH₂CH₂CH₂SAc); ¹³C NMR (100 MHz, CD₃Cl) δ= 170.27, 170.12, 169.53, 169.36, 137.74, 128.71, 128.10, 128.06, 126.62, 126.21, 126.06, 101.40, 100.59, 98.57, 75.70, 74.52, 71.05, 70.85, 70.74, 70.54, 70.52, 70.36, 70.06, 69.99, 69.27, 68.81, 67.23, 66.96, 63.13, 61.25, 18.12, 30.60, 29.32, 29.08, 28.98, 25.33, 23.39, 20.68, 20.53. FAB MS m/z: 1048.2 (MH⁺). Anal: Calcd. for C₄₈H₇₃NO₂₂S: C, 55.00; H, 7.02; N, 1.34; O, 33.58; S, 3.06. Found: C 54.57; H 7.06; N 1.43

### 126E-190-HI

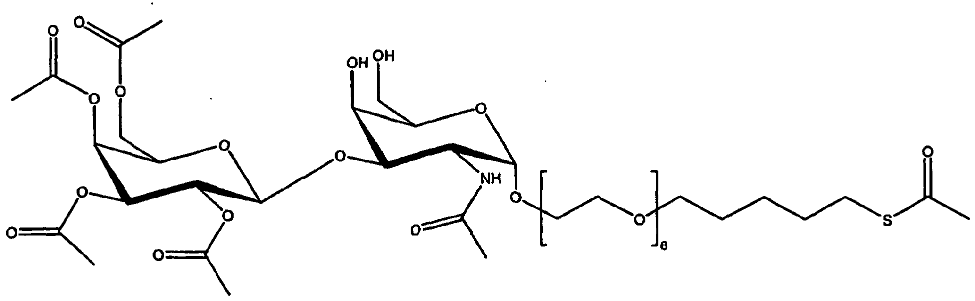

A solution of 1.2g (1.14 mmols) of 126E-188-HI in 20 ml of dry MeOH was treated with a few drops of AcCI for 1.5 hours, quenched with pyridine, evaporated and purified by FCC with 20:1 -> 10:1 CH2Cl2:MeOH to afford 700mg (65%) of 126E-190-HI as a glassy solid R_{f} (10:1 CH₂Cl₂:MeOH)= ; [α]_{D} = +47.57 (c 0.60 in MeOH); ¹H NMR (400 MHz, CD₃Cl) δ= 5.90 (d, 1H, J = 9.74 Hz, NH), 5.37 (m, 1H, H-4"), 5.20 (dd, 1H, J = 7.88, 10.66 Hz, H-2"), 5.00 (m, 1H, H-3"), 4.86 (d, 1H, J = 3.71 Hz, H-1'), 4.66 (d, J = 8.34 Hz, H-1"), 4.57 (m, 1H, H-2'), 3.76-4.22 (m, 7H, H-3', H-6', H-6", H-5', H-5"), 3.54-3.72 (m, 24H, PEG), 3.45 (t, 2H, J = 6.49 Hz, OCH₂CH₂CH₂CH₂CH₂SAc), 2.86 (m, 2H, OCH₂CH₂CH₂CH₂CH₂SAc), 2.32 (s, 3H, NHAc), 2.16 (s, 3H, SAc), 2.08, 2.06, 1.99, 1.98 (s, 12H, 4xAc), 1.59 (m, 4H, OCH₂CH₂CH₂CH₂CH₂SAc), 1.42 (m, 2H, OCH₂CH₂CH₂CH₂CH₂SAc); ¹³C NMR (100 MHz, CD₃Cl) δ= 195.90, 170.36, 170.17, 170.06, 169.59, 169.35, 101.63, 98.15, 78.10, 71.04, 70.77, 70.64, 70.56, 70.53, 70.48, 70.44, 70.20, 70.04, 69.97, 69.81, 69.10, 68.58, 67.05, 66.94, 62.51, 61.28, 47.81, 30.59, 29.31, 29.05, 28.96, 25.32, 23.30, 20.64, 20.60, 20.57, 20.50. FAB MS m/z: 961.4 (MH⁺). Anal: Calcd. for C₄₁H₆₉NO₂₂S: C, 51.29; H, 7.24; N, 1.46; Found: C 50.47; H 6.91; N 1.55.

### 126F-004

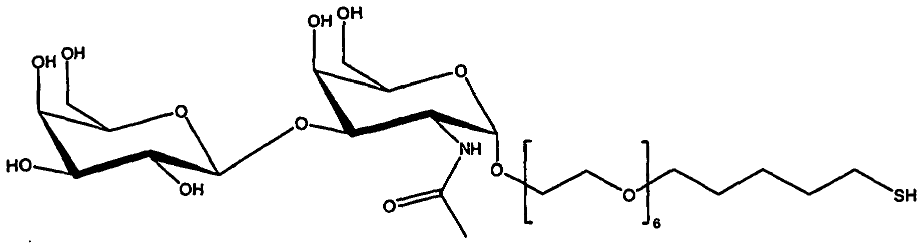

A solution of 500mg of **126E-190-HI** (0.52 mmols) in 20ml of dry MeOH was treated with 7 drops of 25% w/v NaOMe/MeOH and stirred at room temperature for an hour. Reaction was quenched with Amberlite^{®}-120 ion-exchange resin, filtered and evaporated. The residue was cleanly purified with 20% MeOH in CH₂Cl₂ to give 350mg (90%) of **126F-004** as a glassy solid (after liophylization). R_{f} (20% MeOH in CH₂Cl₂)= 0.20; [α]_{D} = +24.36 (c 0.24 in MeOH); ¹H NMR (400 MHz, D₂O) δ= 4.80 (d, 1H, J = 3.71 Hz, H-1'), 4.35 (d, 1H, J = 7.88 Hz, H-1"), 4.24 (dd, 1H, J = 3.71, 10.66 Hz, H-2'), 4.12 (m, 1H, H-4"), 3.94 (dd, 1H, J = 2.78, 11.13 Hz, H-3"), 3.48-3.90 (m, 33H, 24H PEG, NH, H-3', H-4', H-5', H-5", H-6', H-6"), 3.43 (m, 3H, OCH₂CH₂CH₂CH₂CH₂SH, H-2"); 2.44 (m, 2H, OCH₂CH₂CH₂CH₂CH₂SH), 1.91 (s, 3H, NHAc), 1.50 (m, 4H, OCH₂CH₂CH₂CH₂CH₂SH), 1.33 (m, 2H, OCH₂CH₂CH₂CH₂CH₂SH); ¹³C NMR (100 MHz, D₂O) δ= 174.44, 101.75, 97.42, 97.30, 77.25, 74.92, 72.51, 70.87, 70.71, 70.57, 69.57, 69.11, 68.78, 68.69, 68.59, 68.48, 66.40, 61.16, 60.91, 48.45, 32.75, 27.98, 24.02, 23.66, 22.06, 22.04; FAB MS m/z: 750.3 (MH⁺), 788.3 (M+K⁺). Anal: Calcd. for C₃₁H₅₉NO₁₇S: C, 49.65; H, 7.93; N, 1.87. Found: C 49.28; H 7.86; N 1.82.

### 126F-062

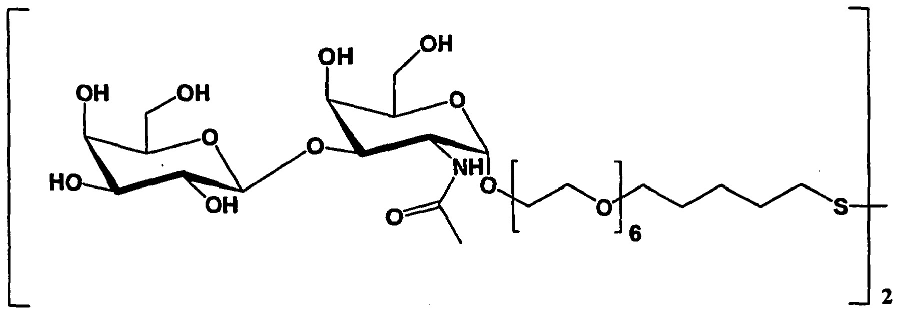

Thiol **126F-004** dimerized on storage into disulfide **126F-062** which was separated from thiol by fcc with 20 → 30% MeOH in CH₂Cl₂. R_{f} = 0.15 (30% MeOH in CH₂Cl; ¹H NMR (400 MHz, D₂O) δ= 4.80 (d, J = 3.81 Hz, H-1'), 4.34 (d, J = 7.61 Hz, H-1"), 4.24 (m, 1H, H-2'), 4.12 (m, 1H, H-4"), 3.94 (dd, 1H, J_{3,4} = 2.93, J_{2,3} = 11.12 Hz, H-3"), 3.48-3.90 (m, 33H, 24H PEG, NH, H-3', H-4', H-5', H-5", H-6', H-6"), 3.43 (m, 3H, OCH₂CH₂CH₂CH₂CH₂SH, H-2"); 2.66 (m, 2H, OCH₂CH₂CH₂CH₂CH₂SH), 1.91 (s, 3H, NHAc), 1.62 (m, 2H, OCH₂CH₂CH₂CH₂CH₂SH), 1.51 (m, 2H, OCH₂CH₂CH₂CH₂CH₂SH), 1.34 (m, 2H, OCH₂CH₂CH₂CH₂CH₂SH); ¹³C NMR (100 MHz, D₂O) δ= 174.41, 104.71, 97.43, 97.31, 77.33, 77.27, 74.90, 72.51, 70.81, 70.69, 70.56, 69.61, 69.22, 68.78, 68.67, 68.58, 68.48, 66.39, 61.14, 60.89, 48.44, 38.19, 28.23, 24.23, 22.06, 22.04; FAB MS m/z: xxxx.x (MH⁺). Anal: Calcd. for C₆₂H₁₁₆N₂O₃₄S₂: C, 49.72; H, 7.81; N, 1.87; Found: C xx.xx; H x.xx; N xx.xx.

### 126F-005

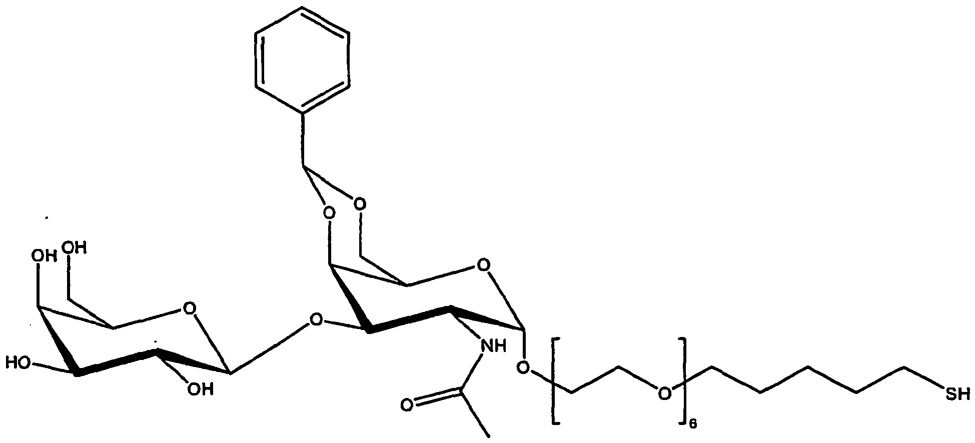

Compound **126F-005** was further debenzylidenated as described for **126E-190-HI** to give additional **126F-004.**

### Phase-transfer preparation of TF-Encapsulated Gold Nanoparticles (TF-AuNP)

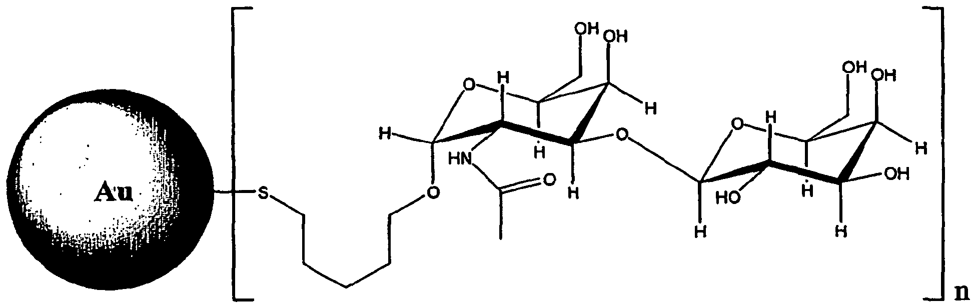

A solution of HAuCl₄ (0.015 mmols) in 1 ml of H₂O was combined with a solution of tetraoctylammonium bromide (0.035 mmols) in 1 ml of toluene. The biphasic mixture was vigorously shaken until all HAuCl₄ was transferred into the toluene layer (orange color). The organic layer was separated. Sodium borohydride (2 mg, 0.053 mmols) was dissolved in the freshly prepared solution of **126B-177** (12.8 mg, 0.013 mmols) in 1 ml of MeOH. Ligand to gold ratio was 1.76:1. The solution of ligand was added dropwise to the toluene solution of HAuCl₄ with vigorous stirring. This led to an immediate formation of dark brown precipitate. After stirring for 1 hour at ambient temperature the nanoparticles were isolated by a few rounds of centrifugation/decantation in methanol. Solution of nanoparticles in water was subjected to SEC on P-4 BioGel® column (exclusion limits ) to remove traces of low MW impurities. This procedure consistently gave high purity nanoparticles as judged by the absence of any sharp peaks in the NMR spectrum. In contrast, separation by centrifugal filtration on Centriplus^{®} YM-30 (MWCO 30,000) always led to small impurities of glycerol which is used by Millipore, Inc. as a humectant of the filter membranes. The lyophilized nanoparticles were separated as a dark powder which was readily soluble in water to give intense purple color (λₘₐₓ = 522 nm). Dilute frozen solutions did not retain the purple coloration but once melted the color. NMR spectrum of the nanoparticles in D₂O closely corresponds to the positions of the functional groups of the ligand **(****Figure 1**). IR (powder) cm⁻¹ 3298.6**4**, 2925.48,2854.13,1711.51, 1619.91.

### Alternative "aqueous" preparation of TF-AuNP (Otsuka H, Akiyama Y, Nagasaki Y, Kataoka K, J. Am. Chem. Soc. 123 (34): 8226-8230 AUG 29 2001)

A solution of 1% HAuCl₄ (1.9 ml, 0.046 mmols in Au³⁺) was added to 200 ml of degassed H₂O. To this solution was added 10mg (0.010 mmols) of TF-S-S-TF (0.020 mmols per thiol). A cooled (0 °C) solution of 20 mg (0.53 mmols) in 25 ml H₂O was added dropwise to the reaction solution with rapid stirring. Resulting colored solution was stirred for additional hour, purified on Millipore's Centriplus^{®} YM-30 centrifugal device and freeze-dried. Ratio of reactants: TFSH:Au = 1:2.3

### Alternative "aqueous" preparation of TF-HEXPEG-AuNP {Otsuka, Akiyama, et al. 2001 324 lid}

A solution of 15 mg (0.02 mmols) of **126F-004** in 1.9 ml (1%) HAuCl₄ and 200 ml of degassed distilled water (Sigma) at 0 °C was slowly treated with a solution of 20 mg NaBH₄ in 25 ml of ice-chilled water. The resulting wine-red solution was stirred overnight, purified on Millipore's Centriplus^{®} YM-30 centrifugal device and freeze-dried. Ratio of reactants: 126F-004: Au³⁺ = 1:2.3. See Table I, "National Cancer Institute".

### Alternative "aqueous" preparation of TF-HEXPEG-AuNP via disulfide {Otsuka, Akiyama, et al. 2001 324 /id}

A solution of 16 mg (0.021 mmols per ligand) of 126F-062 in 1.8 ml of 1% HAuCl₄ was added to 200 ml of ice-chilled degassed distilled water (Sigma). The solution was then slowly treated with 20mg/25ml of ice-chilled NaBH₄ in water. After 20 min at 0 °C and 30 min at ambient temperature the wine-red reaction solution was purified on Millipore's Centriplus^{®} YM-50 centrifugal device and freeze-dried. Ratio of reactants TF-HEXPEG-SH:Au³⁺ = 1:2.3.

### Preparation of TFB-AuNP

### 126E-17

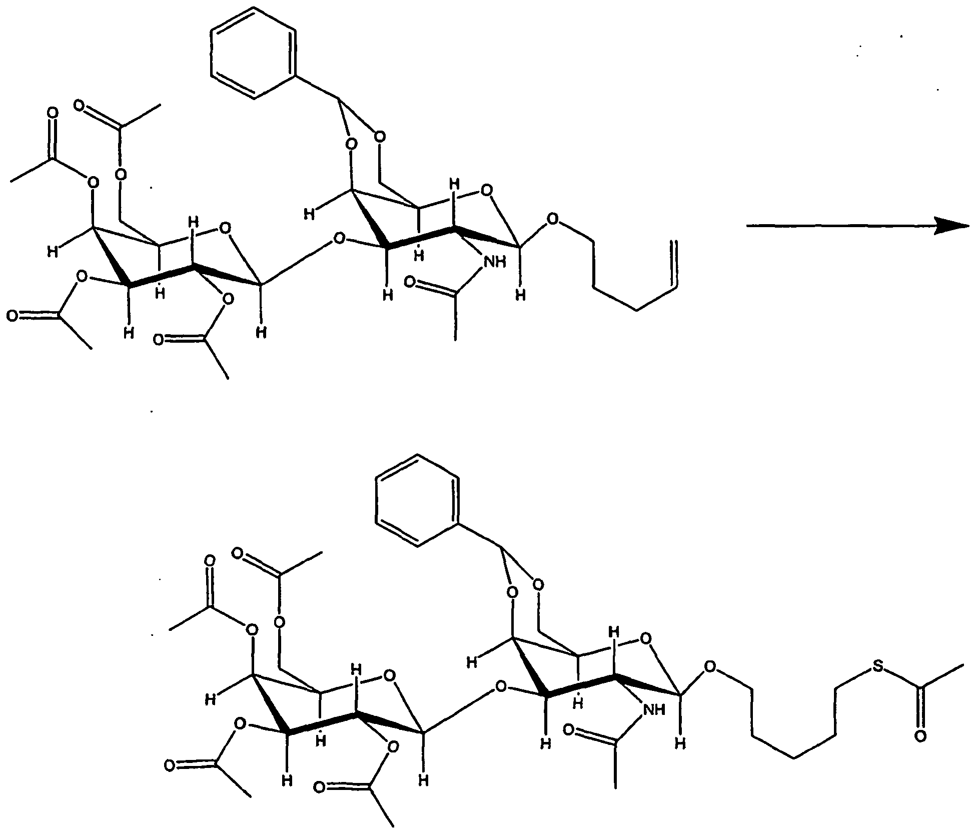

A solution of 660 mg (0.93 mmols) of 126D-14{Svarovsky & Barchi 2003 281 **/id}** in 10 ml of anhydrous 1,4-dioxane was purged with argon for 20 min. To this deoxygenated solution 1.0 ml (13 mmols, 14 eqiuvs) of doubly distilled thiolacetic acid was added followed by 200 mg (1.2 mmols) of AIBN. The reaction was left to stir under argon atmosphere at 75 °C overnight or until no starting material could be detected by TLC. The reaction was then quenched with 1 ml of cyclohexene. The solution was co-evaporated with xylenes under reduced pressure. FCC on silica gel with 6:1 EtOAc/ hexanes provided 655mg (90%) of a glassy solid. R_{f} 0.24 (6:1 EtOAc/hexanes); [α]_{D} = +20.02 (c 2.41 in CHCl₃); IR (neat) 1749.12, 1686.44, 1655.59, 1557.24; ¹H NMR (400 MHz, CDCl₃) δ = 7.47-7.52 (m, 2H, Ph), 7.27-7.35 (m, 3H, Ph), 5.93 (d, 1H, J=6.64Hz, NH), 5.51 (s, 1H, PhCH), 5.32 (d, 1H, J=3.51Hz, H"-4), 5.17 (dd, 1H, J=8.20, 10.15Hz, H"-2), 5.07 (d, 1H, J=8.20Hz, H'-1), 4.95 (m, 1H, H"-3), 4.73 (d, 1H, J=7.81Hz, βH"-1), 4.69 (m, 1H, H'-3), 4.26 (1H, J=12.49Hz, H-6a), 4.25 (m, 1H, H'-4), 4.09 (d, 2H, H-6), 4.01 (d, 1H, J=12.49Hz, H-6b), 3.81-3.90 (m, 2H, -OCH₂CH₂CH₂CH₂CH₂SAc, H"-5), 3.30-3.45 (m, 3H, -OCH₂CH₂CH₂CH₂CH₂SAc, H'-5, H'-2), 2.73-2.86 (m, 2H, - OCH₂CH₂CH₂CH₂CH₂SAc), 2.26 (s, 3H, SAc), 2.10 (s, 3H, NHAc), 1.99, 1.98, 1.93, 1.92 (s, 12H, OAc), 1.48-1.60 (m, 4H, -OCH₂CH₂CH₂CH₂CH₂SAc), 1.32-1.40 (m, 2H, - OCH₂CH₂CH₂CH₂CH₂SAc); ¹³C NMR (100 MHz, CD₃Cl) δ = 196.42, 171.25, 170.49, 170.28, 169.32, 138.05, 128.99, 128.27, 126.40, 101.47, 100.85, 99.11, 76.19, 75.77, 71.24, 71.05, 69.63, 69.52, 69.26, 67.30, 66.63, 61.86, 54.81, 30.84, 29.55, 29.20, 29.01, 25.52, 23.92, 21.03, 20.88, 20.74. FAB MS m/z: 784.1 (MH⁺). Anal: Calcd. for C₃₆H₄₉NO₁₆S: C 55.16; H 6.30; N 1.79. Found: C 54.31; H 6.08; N 1.81.

### 126E-20

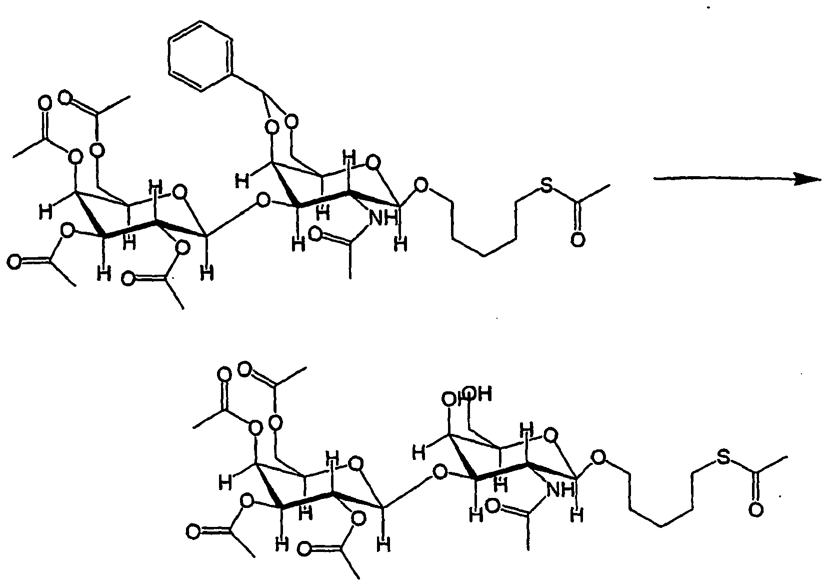

A solution of 600 mg (0.76 mmols) of **126E-17** in 15 ml of methanol was treated with a few drops of acetyl chloride and the reaction was stirred at room temperature for an hour. The reaction was quenched with 1 ml of pyridine and concentrated. Purification of the crude by fcc with 5% to 10% MeOH in CH₂Cl₂ yielded 510 mg (96%) of **126E-20** as a glassy solid. R_{f} 0.60 (10% MeOH in CH₂Cl₂); [α]_{D} = +9.55 (c 1.69 in CHCl₃); IR (neat) 3565.74, 3283.21, 1751.05, 1735.62, 1696.09, 1652.70; ¹H NMR (400 MHz, CDCl₃) δ = 6.05 (d, 1H, J=7.03Hz, NH), 5.33 (d, 1H, J=3.12Hz, H"-4), 5.13 (m, 1H, **H"-2),** 4.98 (dd, 1H, J=3.51, 10.54Hz, H"-3), 4.91 (d, 1H, J=8.59Hz, βH'-1), 4.61 (d; 1H, J=7.81Hz, βH"-1), 4.56 (m, 1H, H'-3), 4.03 (m, 1H, H'-4), 3.82 (m, 1H, -OCH₂CH₂CH₂CH₂CH₂SAc), 4.14-3.70 (m, 5H, H'-6, H"-6, H-5), 3.57 (m, 1H, H-5), 3.42 (m, 1H, -OCH₂CH₂CH₂CH₂CH₂SAc), 3.24 (m, 1H, H'-2), 2.86 (s, 1H, 4-OH), 2.78 (m, 2H, -OCH₂CH₂CH₂CH₂CH₂SAc), 2.58 (s, 1H, 6-OH), 2.27 (s, 3H, SAc), 2.11 (s, 3H, NHAc), 2.02, 2.01, 1.93 (s, 12H, OAc), 1.48-1.58 (m, 4H, - OCH₂CH₂CH₂CH₂CH₂SAc), 1.30-1.40 (m, 2H, -OCH₂CH₂CH₂CH₂CH₂SAc); ¹³C NMR (100 MHz, CD₃Cl) δ = 196.55, 171.04, 170.74, 170.33, 170.22, 169.61, 101.43, 99.35, 78.66, 73.91, 71.20, 70.86, 69.69, 68.93, 68.43, 67.20, 62.61, 61.78, 54.69, 30.84, 29.50, 29.15, 29.01, 25.44, 23.87, 20.95, 20.82, 20.78, 20.72. FAB MS m/z: 696.1 (MH⁺). Anal: Calcd. for C₂₉H₄₅NO₁₆S: C, 50.06; H, 6.52; N, 2.01; Found: C 49.41; H 6.52; N 2.04.

### 126E-23

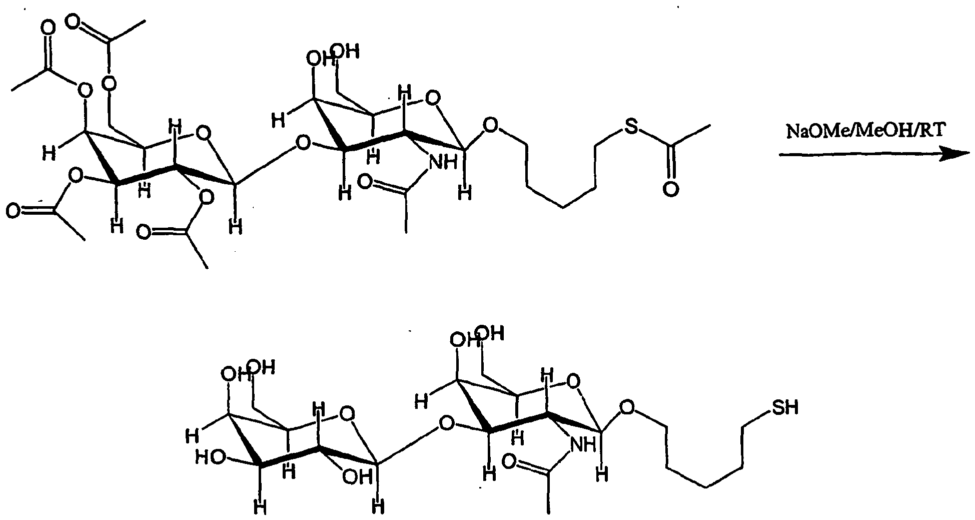

A solution of 300 mg (0.43 mmols) of **126E-20** in 5 ml of MeOH was treated with 5 drops of 25% (w/v) NaOMe/MeOH. The reaction was stirred at RT for 10 minutes. The reaction was carefully neutralized with strongly acidic Amberlite^{®}-120, and evaporated under reduced pressure at 50°C to give 200 mg (96%) of **126E-23** contaminated by small amount of dimeric disulfide. The crude product was purified by RPFC with 10% → 40% (v/v) MeOH/H₂O to give 187 mg (90%) of **126E-23** as a white powder. The compound was soluble in water and moderately soluble in methanol and chloroform. R_{f} 0.10 (20% MeOH-CH₂Cl₂); [α]_{D} = -6.48 (c 0.50 in MeOH); IR (neat) 3274.54, 1617.02, 1563.99; ¹H NMR (400 MHz, D₂O) δ = 4.37 (d, 1H, J = 8.59 Hz, βH-1"), 4.31 (d, 1H, J = 7.42 Hz, βH-1'), 4.04 (d, 1H, J = 2.73 Hz, NH), 3.36-3.90 (m, 14H, H-2", 2xH-6', 2xH-6", H-3', H-3", H-4', H-4", H-5', H-5", - OCH₂CH₂CH₂CH₂CH₂SH, H-2'), 2.41 (m, 2H, -OCH₂CH₂CH₂CH₂CH₂SH), 1.89 (s, 3H, NHAc), 1.39-1.53 (m, 4H, -OCH₂CH₂CH₂CH₂CH₂SH), 1.23-1.32 (m, 2H,-OCH₂CH₂CH₂CH₂CH₂SH); ¹³C NMR (100 MHz, CD₃Cl δ =174.82, 105.01, 101.51, 80.18, 80.03, 75.23, 75.08, 74.98, 74.83, 72.71, 72.57, 70.83, 70.53, 70.37, 70.21, 68.84, 68.67, 68.28, 68.15, 61.25, 61.18, 61.09, 51.43, 32.77, 28.20, 24.09, 23.86, 22.53, 22.44.FAB MS m/z: 486.0 (MH⁺), 508.1 (M+Na⁺). Anal: Calcd. for C₁₉H₃₅NO₁₁S: C, 47.00; H, 7.27; N, 2.88; Found: C 44.09; H 7.14; N 2.76.

### In Vivo Immunotherapy Mice Experiments and Results

### In vivo Anti-Tumor Effect of JAA-F11

To investigate the anti-tumor effect of JAA-F11 antibody in vivo, the 4T1 mouse breast cancer model was used. The 4T1 tumor line has several characteristics that make it a suitable experimental animal model for human mammary cancer. First, 4T1 tumor cells are TAg, positive, easily transplanted into the mammary gland so that the primary tumor can grow in situ, and metastases readily developed. The primary tumor can be surgically removed, so that metastatic disease can be studied in an animal model, comparable to the clinical situation where the primary breast tumor is surgically removed and metastatic foci remain intact (Pulaski et al, 1998, Pulaski et al, 2000, Aslakson et al, 1992).

### Maintaining and harvesting 4T1 Tumor Cells in vitro

4T1 tumor cells were cultured in a 250 ml polystyrene vented culture flask with RPMI 1640 medium containing 2mM L-glutamine, 1.5g/L sodium bicarbonate, 4.5 g/L glucose, 10mM HEPES and 1.0mM sodium pyruvate (90%) plus 10% FBS. The culture flasks were incubated in a 37 °C, 5% CO2 tissue culture incubator. Cultures were split 2 to 3 times per week and were not grown in vitro longer than one month before implantation. The cells were not allowed to exceed 50% to 80% confluence as overgrowth will decrease the viability of cells. When the cells were harvested, the culture medium was discarded. Five ml sterile PBS was added to wash the flask and discarded. Five ml trypsin solution (Trypsin-0.25%, EDTA-0.1 %) solution was added to cover the side of the flask and was incubated at 37°C for 5 minutes. Twenty-five ml of medium was added to harvest trypsinized cells from flask and transferred to a 50- ml conical tube. The tube with harvested cells was centrifuged 10 min at 1000 rpm. The supernatant was discarded and the cell pellet was resuspended in 10 ml of medium. A 30cc syringe with an 18-G needle was used to separate 4T1 cells into single-cell suspension. The viable cell concentration was determined using a hemacytometer. The cells were diluted with the same medium to the desired concentration for injection.

### In vivo experiments

According to previously published studies (Pulaski et al, 1998, Pulaski et al, 2000, Aslakson et al, 1992), 1*10⁴ viable tumor cells per mouse was selected to ensure the tumor incidence will be 100% but the tumor load will be not too great for Ab treatment.

### Injecting mice with 4T1 tumor cells and T-Ag gold or PBS

One hundred microliters of 10⁵ viable cells/ml 4T1 cell suspension was subcutaneously inoculated in the right abdominal mammary gland of eight-week-old female BALB/c mice. Three days after implanting the 4T1 tumor cells, twenty five mice were divided into two groups randomly, and received an intraperitoneal administration of either purified T Ag gold (5mice) (5µg/100µl/mouse) as treatment or PBS (20 mice) as control twice weekly. On the fourteenth day after implanting 4T1 tumor, the primary breast tumors were surgically removed.

### Measurement of tumor outcome

The mouse weights were measured and the weight loss rates were compared between the treated and the control mice. Clinical symptoms, such as lack of grooming, rough coat, rapid and labored breathing, and loss of mobility were monitored and recorded and used as indicators of morbidity. Mice were sacrificed when the tumor diameter reached 14 mm or weight loss reached 20% or significant morbidity occurred. Daily observations were made by both investigators and animal caretakers. The survival time was recorded and analyzed by Kaplan-Meier Survival Curve (MedCalc). The organs of interest (primary tumor, lung, liver, spleen, lymph node and brain) were collected and fixed using Z-fixative. Number of metastasis grossly apparent in the lungs were counted. Difference in the number of metastasis was analyzed by Chi- Square analysis using MedCalc Software Version 7.2. Paraffin sections for immunohistochemical staining were prepared for histological analyses of metastasis presence.

### Analysis of Results

The median survival time of PBS and T-Ag groups was days 57 and 70 respectively. The difference was not significant (p > 0.05) according to the results of Kaplan-Meier Curve. This is being repeated with a larger number of animals.

The organs of interest, such as primary tumor, lung, liver, brain, spleen, were collected. Metastatic lesions were found on lymph nodes, ribs, pericardium and lungs in mice from both groups. The levels of metastases on lungs were classified into four groups after counting the number of metastatic lesions grossly visible in the lungs as shown in the Figures. The mice with TF Ag treatment had less metastasis on the lungs compared to those with PBS control. Two sets of the lungs from the T-ag gold group that were grossly negative for tumor metastasis were examined for micrometastasis by a pathologist in a blinded study with several lungs with metastatic lesions. Of the two mice in the PBS control group, "no metastases observed", one had histologically apparent tumor nodules, while the other did not.

These results indicate that gold nanoparticles coated with a specific tumor associated carbohydrate antigen; one that is displayed in 90% of human carcinomas to the immune system during tumor growth and progression. TF antigen is an O-linked glycoprotein is ideal for studying the interactions that may be occurring *in vivo* when a tumor cell metastasizes. A breast cancer model cell line, 4T1, was investigated using the TF-Antigen conjugated gold nanoparticles. Without being bound by a particular theory of operation, the 4T1 cell line appears to metastasize through interactions with the TF disaccharide that was used to prepare the gold nanoparticle conjugates. In addition, TF-antigen disaccharide appears to be involved in a specific carbohydrate-protein interaction that aids lung colonization of 4T1 breast cancer cells. Tumors were first implanted in mice and then allowed to grow. Treatment with nanoparticle conjugates began on the third day after implantation. When the primary tumors were palpable, they were surgically removed and the mice were continued to be treated with the nanoparticle conjugates. The TF-antigen gold nanoparticle conjugates inhibited metastases from the metastatic site in the lungs. Without being bound by a particular theory of operation, Ab to TF Ag apparently can either increase or decrease proliferation rate, as can lectins that bind TF Ag. Apparently, the addition of a TF-antigen containing gold particle may be able to interfere with a process involved in replication of the tumor cell, in addition to being able to inhibit adhesion. In addition, TF-antigen gold containing nanoparticle conjugates may also have utility in the elucidation of the mechanism of TF-antigen and replication.

## Claims

1. A method of preparing antigen-nanoparticle conjugates, comprising:
providing a nanoparticle; and
conjugating a plurality of carbohydrate antigens to the nanoparticle, wherein the carbohydrate antigens include TF antigen.

2. The method of claim 1, wherein the plurality of carbohydrate antigens further includes Tₙ antigen, Gb1 antigen, GM₁ antigen, GM₃ antigen, Lewis Y Antigen, or any combination thereof.

3. The method of claim 1 or 2, wherein a plurality of nanoparticles are provided, and a plurality of carbohydrate antigens are conjugated to at least a portion of the nanoparticles.

4. The method of claim 3, wherein a plurality of carbohydrate antigens are conjugated to each of the nanoparticles.

5. The method of claim 1 or 2, wherein the carbohydrate antigens comprise a linking group, and the linking group is conjugated to the nanoparticle.

6. The method of claim 1 or 2, wherein conjugating includes self-assembly of the carbohydrate antigens on at least one surface of the nanoparticle.

7. The method of claim 1 or 2, wherein the nanoparticle comprises one or more nanoparticle linking groups, and the carbohydrate antigens are conjugated to the nanoparticle linking groups.

8. The method of claim 1 or 2, wherein one or more spacer groups link the carbohydrate antigens to the nanoparticle.

9. The method of claim 8, wherein the one or more spacer groups include PEG, a carbon chain, a carbon chain including sulfur, nitrogen or oxygen in the backbone, polymers including polyacrylamide, a peptide chain made up of all glycine or alanine units, or any combination thereof.

10. The method of claim 1 or 2, further comprising purifying the nanoparticles.

11. The method of claim 10, wherein said purifying includes filtration, centrifugation, electrophoresis, chromatography, crystallization, or any combination thereof.

12. An antigen-nanoparticle conjugate comprising a plurality of carbohydrate antigens conjugated to a nanoparticle, wherein the antigens include TF antigen.

13. The antigen-nanoparticle conjugate of claim 12, wherein the antigens further include Tₙ antigen, Gbl antigen, GM₁ antigen, GM₃ antigen, Lewis Y Antigen, or any combination thereof.

14. The antigen-nanoparticle conjugate of claim 12 or 13, wherein the plurality of carbohydrate antigens are identical to each other.

15. The antigen-nanoparticle conjugate of claim 12 or 13, wherein at least a portion of the carbohydrate antigens are displayed to the immune system in human carcinomas during tumor growth and progression.

16. The antigen-nanoparticle conjugate of claim 12 or 13, wherein the carbohydrate antigens are capable of inducing galectin-3 surface expression in endothelial cells.

17. The antigen-nanoparticle conjugate of claim 12 or 13, wherein at least one of the plurality of tumor-associated carbohydrate antigens is conjugated to a spacer group, and the spacer group is conjugated to the nanoparticle.

18. The antigen-nanoparticle conjugate of claim of 12 or 13, wherein the spacer group includes PEG, a carbon chain, a carbon chain including sulfur, nitrogen or oxygen in the backbone, a polymer, a peptide, or any combination thereof.

19. The antigen-nanoparticle conjugate of claim 12 or 13, wherein one or more carbohydrate antigens are conjugated to one or more linking groups, and the linking groups are conjugated to the nanoparticle.

20. The antigen-nanoparticle conjugate of claim 12 or 13, wherein one or more carbohydrate antigens are conjugated to one or more spacer groups, the spacer groups are conjugated to one or more linking groups, and the one or more linking groups are conjugated to the nanoparticle.

21. The method of claim 4 or antigen-nanoparticle conjugate of claim 19 or 20 wherein the linking groups include at least one sulfur atom, carboxylate group, amide group, carbamate group carbonate group, thiocarbamate group, thiocarbonate group, thioether group, succinimide group, n-hydroxy succinimide group, or any combination thereof.

22. The antigen-nanoparticle conjugate of claim 12 or 13, wherein each of the carbohydrate antigens are covalently linked to one or more sulfur atoms.

23. The antigen-nanoparticle conjugate of claim 12 or 13, wherein the carbohydrate antigens are each linked, individually, to one or more sulfur atoms.

24. The antigen-nanoparticle conjugate of claim 12 or 13, wherein each of the carbohydrate antigens include one or more amino acids.

25. The antigen-nanoparticle conjugate of claim 22, wherein at least two sulfur atoms are covalently linked to each other.

26. The antigen-nanoparticle conjugate of claim 22, wherein at least one of the sulfur atoms is bonded to the nanoparticle.

27. The antigen-nanoparticle conjugate of claim 26, wherein the bonds between the sulfur atoms and the nanoparticle include covalent bonds, hydrogen bonds, ionic bonds, van der Waals bonds, or any combination thereof.

28. The antigen-nanoparticle conjugate of claim 12 or 13, wherein the carbohydrate antigens include a disaccharide.

29. The antigen-nanoparticle conjugate of claim 28, wherein the disaccharide comprises at least one amino sugar group.

30. The antigen-nanoparticle conjugate of claim 12 or 13, wherein at least one of the plurality of the carbohydrate antigens is a prognostic indicator for cancer, a marker of metastasized carcinoma cells, an adhesion molecule involved in metastasis, or any combination thereof.

31. The antigen-nanoparticle conjugate of claim 12 or 13, wherein the nanoparticle includes gold atoms, silver atoms, platinum atoms, rhodium atoms, palladium atoms, or any combination thereof.

32. The antigen-nanoparticle conjugate of claim 31, wherein the nanoparticle is derived from colloidal gold.

33. The antigen-nanoparticle conjugate of claim 12 or 13, wherein the antigen-nanoparticle conjugate has a molecular weight in the range of from about 1,000 Daltons to about 1 million Daltons.

34. The antigen-nanoparticle conjugate of claim 12 or 13, wherein the number of carbohydrate antigens conjugated to the nanoparticle is in the range of from about 2 to about 1000.

35. The antigen-nanoparticle conjugate of claim 12 or 13, wherein the nanoparticle has from about 50 to about 10,000 atoms.

36. The antigen-nanoparticle conjugate of claim 12 or 13, wherein the nanoparticle has a dimension in the range of from about 0.5 nm to about 100nm.

37. The antigen-nanoparticle conjugate of claim 36, wherein the nanoparticle has a dimension in the range of from about 1 nm to about 10nm.

38. The antigen-nanoparticle conjugate of claim 36, wherein the nanoparticle has a spheroidal shape, and the diameter of the nanoparticle is in the range of from about 1 nm to about 10nm.

39. The antigen-nanoparticle conjugate of claim 36, wherein the nanoparticle has a icosahedral, cubic, rhombic, hexagonal, or fullerene shape.

40. The antigen-nanoparticle conjugate of any one of claims 12-39 for use in therapy.

41. Use of the antigen-nanoparticle conjugate of any one of claims 12-39 for the manufacture of a medicament for inhibiting metastasis of carcinoma cells in a mammal.

42. The use of claim 41, wherein the mammal is a human.

43. The use of claim 42, wherein the human is diagnosed as having cancer.

44. The use of claim 43, wherein the cancer is breast cancer.

45. The use of claim 44, wherein metastasis to lung cells is inhibited.

46. The use of claim 41, further comprising removing tumor cells from said mammal.

47. The use of claim 46, wherein the tumor cells are removed from said mammal prior to administering the therapeutically effective amount of the antigen-nanoparticle conjugates.

48. The use of claim 41, wherein the carbohydrate antigens are displayed to the immune system in human carcinomas during tumor growth and progression.

49. The use of claim 41, wherein the carbohydrate antigens are capable of inducing galectin-3 surface expression in endothelial cells.

50. The use of claim 41, wherein the carbohydrate antigens are conjugated to an exterior surface of said nanoparticle.

51. The use of claim 41, wherein at least one of the tumor-associated carbohydrate antigens is conjugated, individually, to at least one spacer group, and the at least one spacer group is conjugated to the nanoparticle.

52. The use of claim of 51, wherein the spacer group includes PEG, a carbon chain, a carbon chain including sulfur, nitrogen or oxygen in the backbone, a polymer, a peptide, or any combination thereof.

53. The use of claim 41, wherein one or more carbohydrate antigens are conjugated to a linking group.

54. The use of claim 41, wherein one or more carbohydrate antigens are conjugated, individually to a spacer group, and one or more spacer groups conjugated to a linking group.

55. The use of claim 54, wherein the linking group include at least one sulfur atom, carboxylate group, amide group, carbamate group, carbonate group, thiocarbamate group, thiocarbonate group, thioether group, succinimide group, n-hydroxy succinimide group, or any combination thereof.

56. The use of claim 41, wherein each of the carbohydrate antigens is covalently linked to one or more sulfur atoms.

57. The use of claim 56, wherein at least two of the sulfur atoms are bonded to each other.

58. The use of claim 56, wherein the carbohydrate antigens are each linked, individually, to one or more sulfur atoms.

59. The use of claim 56, wherein at least one of the sulfur atoms is bonded to at least one of the plurality of nanoparticles using NaBH₄.

60. The use of claim 59, wherein the bonds between the sulfur atoms and the nanoparticle are characterized as being covalent bonds, ionic bonds, hydrogen bonds, van der Waals bonds, or any combination thereof.

61. The use of claim 41, wherein the carbohydrate antigens is a prognostic indicator, a marker of metastasized carcinoma cells, an adhesion molecule involved in metastasis, or any combination thereof.

62. The use of claim 41, wherein at least one of the plurality of the nanoparticles includes gold atoms, silver atoms, platinum atoms, rhodium atoms, palladium atoms, or any combination thereof.

63. The use of claim 62, wherein the nanoparticles include colloidal gold particles.

64. The method of claim 1 or 2, wherein the molecular weights of at least a portion of the antigen-nanoparticle conjugates is in the range of from about 1,000 Daltons to about 1 million Daltons.

65. The method of claim 64, wherein the molecular weights of at least a portion of the antigen-nanoparticle conjugates is in the range of from about 10,000 Daltons to about 500,000 Daltons.

66. The use of claim 41, wherein at least a portion of the antigen nanoparticle conjugates comprise, individually, from 2 to about 1000 carbohydrate antigens.

67. The use of claim 41, wherein at least a portion of the nanoparticles comprise from about 50 to about 10,000 atoms.

68. The use of claim 44, wherein at least a portion of the nanoparticles has a dimension in the range of from about 0.5nm to about 100nm.

69. The use of claim 68, wherein at least a portion of the nanoparticles has a dimension in the range of from about 1 nm to about 10nm.

70. The use of claim 68, wherein at least a portion of the nanoparticles comprises a spheroid shape, and the dimension is the diameter of the nanoparticle.

## Patentansprüche

1. Verfahren zur Herstellung von Antigen-Nanoteilchen-Konjugaten, umfassend:
Bereitstellen eines Nanoteilchens; und
Konjugieren einer Mehrzahl von Kohlenhydrat-Antigenen an das Nanoteilchen, wobei die Kohlenhydrat-Antigene TF-Antigen einschließen.

2. Verfahren nach Anspruch 1, wobei die Mehrzahl von Kohlenhydrat-Antigenen weiter Tₙ-Antigen, Gb1-Antigen, GM₁-Antigen, GM₃-Antigen, Lewis-Y-Antigen oder irgendeine Kombination davon einschließt.

3. Verfahren nach Anspruch 1 oder 2, wobei eine Mehrzahl von Nanoteilchen bereitgestellt werden und eine Mehrzahl von Kohlenhydrat-Antigenen an wenigstens einen Teil der Nanoteilchen konjugiert werden.

4. Verfahren nach Anspruch 3, wobei eine Mehrzahl von Kohlenhydrat-Antigenen an jedes der Nanoteilchen konjugiert werden.

5. Verfahren nach Anspruch 1 oder 2, wobei die Kohlenhydrat-Antigene eine Verknüpfungsgruppe umfassen und die Verknüpfungsgruppe an das Nanoteilchen konjugiert wird.

6. Verfahren nach Anspruch 1 oder 2, wobei das Konjugieren den Selbstzusammenbau der Kohlenhydrat-Antigene auf wenigstens einer Oberfläche des Nanoteilchens einschließt.

7. Verfahren nach Anspruch 1 oder 2, wobei das Nanoteilchen eine oder mehrere Nanoteilchen-Verknüpfungsgruppen umfasst und die Kohlenhydrat-Antigene an die Nanoteilchen-Verknüpfungsgruppen konjugiert werden.

8. Verfahren nach Anspruch 1 oder 2, wobei eine oder mehrere Spacergruppen die Kohlenhydrat-Antigene mit dem Nanoteilchen verknüpfen.

9. Verfahren nach Anspruch 8, wobei die eine oder mehrere Spacergruppen PEG, eine Kohlenstoffkette, eine Kohlenstoffkette, die Schwefel, Stickstoff oder Sauerstoff in der Hauptkette einschließt, Polymere, einschließlich Polyacrylamid, eine Peptidkette, die vollständig aus Glycin- oder Alanin-Einheiten besteht, oder irgendeine Kombination davon umfasst.

10. Verfahren nach Anspruch 1 oder 2, das weiter das Reinigen der Nanoteilchen umfasst.

11. Verfahren nach Anspruch 10, wobei das Reinigen Filtration, Zentrifugation, Elektrophorese, Chromatographie, Kristallisation oder irgendeine Kombination davon einschließt.

12. Antigen-Nanoteilchen-Konjugat, umfassend eine Mehrzahl von Kohlenhydrat-Antigenen, die an ein Nanoteilchen konjugiert sind, wobei die Antigene TF-Antigen einschließen.

13. Antigen-Nanaoteilchen-Konjugat nach Anspruch 12, wobei die Antigene weiter Tₙ-Antigen, Gb1-Antigen, GM₁-Antigen, GM₃-Antigen, Lewis-Y-Antigen oder irgendeine Kombination davon einschließen.

14. Antigen-Nanoteilchen-Konjugat nach Anspruch 12 oder 13, wobei die Mehrzahl von Kohlenhydrat-Antigenen identisch zueinander sind.

15. Antigen-Nanoteilchen-Konjugat nach Anspruch 12 oder 13, wobei wenigstens ein Teil der Kohlenhydrat-Antigene dem Immunsystem in menschlichen Karzinomen während des Tumorwachstums und der Tumorprogression präsentiert werden.

16. Antigen-Nanoteilchen-Konjugat nach Anspruch 12 oder 13, wobei die Kohlenhydrat-Antigene in der Lage sind, Galectin-3-Oberflächenexpression in Endothelzellen zu induzieren.

17. Antigen-Nanoteilchen-Konjugat nach Anspruch 12 oder 13, wobei wenigstens eines aus der Mehrzahl von Tumor-assoziierten Kohlenhydrat-Antigenen an eine Spacergruppe konjugiert ist und die Spacergruppe an das Nanoteilchen konjugiert ist.

18. Antigen-Nanoteilchen-Konjugat nach Anspruch 12 oder 13, wobei die Spacergruppe PEG, eine Kohlenstoffkette, eine Kohlenstoffkette, die Schwefel, Stickstoff oder Sauerstoff in der Hauptkette einschließt, ein Polymer, ein Peptid oder irgendeine Kombination davon einschließt.

19. Antigen-Nanoteilchen-Konjugat nach Anspruch 12 oder 13, wobei eines oder mehrere Kohlenhydrat-Antigene an eine oder mehrere Verknüpfungsgruppen konjugiert sind und die Verknüpfüngsgruppen an das Nanoteilchen konjugiert sind.

20. Antigen-Nanoteilchen-Konjugat nach Anspruch 12 oder 13, wobei eine oder mehrere Kohlenhydrat-Antigene an eine oder mehrere Spacergruppen konjugiert sind, die Spacergruppen an eine oder mehrere Verknüpfungsgruppen konjugiert sind und die eine oder mehreren Verknüpfungsgruppen an das Nanoteilchen konjugiert sind.

21. Verfahren nach Anspruch 4 oder Antigen-Nanoteilchen-Konjugat nach Anspruch 19 oder 20, wobei die Verknüpfungsgruppen wenigstens ein Schwefelatom, eine Carboxylatgruppe, eine Amidgruppe, eine Carbamatgruppe, eine Carbonatgruppe, eine Thiocarbamatgruppe, eine Thiocarbonatgruppe, eine Thioethergruppe, eine Succinimidgruppe, eine N-Hydroxysuccinimidgruppe oder irgendeine Kombination davon einschließen.

22. Antigen-Nanoteilchen-Konjugat nach Anspruch 12 oder 13, wobei jedes der Kohlenhydrat-Antigene kovalent mit einem oder mehreren Schwefelatomes verknüpft ist.

23. Antigen-Nanoteilchen-Konjugat nach Anspruch 12 oder 13, wobei die Kohlenhydrat-Antigene, einzeln, jeweils mit einem oder mehreren Schwefelatomen verknüoft sind.

24. Antigen-Nanoteilchen-Konjugat nach Anspruch 12 oder 13, wobei jedes der Kohlenhydrat-Antigene eine oder mehrere Aminosäuren einschließt.

25. Antigen-Nanoteilchen-Konjugat nach Anspruch 22, wobei wenigstens zwei Schwefelatome kovalent miteinander verknüpft sind.

26. Antigen-Nanoteilchen-Konjugat nach Anspruch 22, wobei wenigstens eines der Schwefelatome an das Nanoteilchen gebunden ist.

27. Antigen-Nanoteilchen-Konjugat nach Anspruch 26, wobei die Bindungen zwischen den Schwefelatomen und dem Nanoteilchen kovalente Bindungen, Wasserstoffbrückenbindungen, ionische Bindungen, van-der-Waals-Bindungen oder irgendeine Kombination davon einschließen.

28. Antigen-Nanoteilchen-Konjugat nach Anspruch 12 oder 13, wobei die Kohlenhydrat-Antigene ein Disaccharid einschließen.

29. Antigen-Nanoteilchen-Konjugat nach Anspruch 28, wobei das Disaccharid wenigstens eine Aminozuckergruppe umfasst.

30. Antigen-Nanoteilchen-Konjugat nach Anspruch 12 oder 13, wobei wenigstens eines aus der Mehrzahl der Kohlenhydrat-Antigene ein Prognoseindikator für Krebs, ein Marker für metastasierte Karzinomzellen, ein Adhäsionsmolekül, das bei Metastase involviert ist, oder irgendeine Kombination davon ist.

31. Antigen-Nanoteilchen-Konjugat nach Anspruch 12 oder 13, wobei das Nanoteilchen Goldatome, Silberatome, Platinatome, Rhodiumatome, Palladiumatome oder irgendeine Kombination davon einschließt.

32. Antigen-Nanoteilchen-Konjugat nach Anspruch 31, wobei das Nanoteilchen von kolloidalem Gold abgeleitet ist.

33. Antigen-Nanoteilchen-Konjugat nach Anspruch 12 oder 13, wobei das Antigen-Nanoteilchen-Konjugat ein Molekulargewicht im Bereich von etwa 1.000 Daltons bis etwa 1 Millionen Daltons besitzt.

34. Antigen-Nanoteilchen-Konjugat nach Anspruch 12 oder 13, wobei die Anzahl von Kohlenhydrat-Antigenen, die an das Nanoteilchen konjugiert sind, im Bereich von etwa 2 bis etwa 1000 liegt.

35. Antigen-Nanoteilchen-Konjugat nach Anspruch 12 oder 13, wobei das Nanoteilchen von etwa 50 bis etwa 10.000 Atome besitzt.

36. Antigen-Nanoteilchen-Konjugat nach Anspruch 12 oder 13, wobei das Nanoteilchen eine Abmessung im Bereich von etwa 0,5 nm bis etwa 100 nm besitzt.

37. Antigen-Nanoteilchen-Konjugat nach Anspruch 36, wobei das Nanoteilchen eine Abmessung im Bereich von etwa 1 nm bis etwa 10 nm besitzt.

38. Antigen-Nanoteilchen-Konjugat nach Anspruch 36, wobei das Nanoteilchen eine Kugelform besitzt und der Durchmesser des Nanoteilchens im Bereich von etwa 1 nm bis etwa 10 nm liegt.

39. Antigen-Nanoteilchen-Konjugat nach Anspruch 36, wobei das Nanoteilchen eine Icosaeder-, Würfel-, Rhomben-, Hexagon- oder Fulleren-Form besitzt.

40. Antigen-Nanoteilchen-Konjugat nach einem der Anspruche 12 bis 39 zur Verwendung in der Therapie.

41. Verwendung des Antigen-Nanoteilchen-Konjugats nach einem der Ansprüche 12 bis 39 zur Herstellung eines Arzneimittels zur Hemmung der Metastase von Karzinomzellen in einem Säugetier.

42. Verwendung nach Anspruch 41, wobei das Säugetier ein Mensch ist.

43. Verwendung nach Anspruch 42, wobei bei dem Menschen Krebs diagnostiziert ist.

44. Verwendung nach Anspruch 43, wobei der Krebs Brustkrebs ist.

45. Verwendung nach Anspruch 44, wobei Metastase von Lungenzellen gehemmt wird.

46. Verwendung nach Anspruch 41, die weiter das Entfernen von Tumorzellen aus dem Säugetier umfasst.

47. Verwendung nach Anspruch 46, wobei die Tumorzellen aus dem Säugetier vor der Verabreichung der therapeutisch wirksamen Menge der Antigen-Nanoteilchen-Konjugate entfernt werden.

48. Verwendung nach Anspruch 41, wobei die Kohlenhydrat-Antigene dem Immunsystem in menschlichen Karzinomen während des Tumorwachstums und der Tumorprogression präsentiert werden.

49. Verwendung nach Anspruch 41, wobei die Kohlenhydrat-Antigene in der Lage sind, Galectin-3-Oberflächenexpression in Endothelzellen zu induzieren.

50. Verwendung nach Anspruch 41, wobei die Kohlenhydrat-Antigene an eine äußere Oberfläche des Nanoteilchens konjugiert sind.

51. Verwendung nach Anspruch 41, wobei wenigstens eines von den Tumor-assoziierten Kohlenhydrat-Antigenen, einzeln, an wenigstens eine Spacergruppe konjugiert ist und die wenigstens eine Spacergruppe an das Nanoteilchen konjugiert ist.

52. Verwendung nach Anspruch 51, wobei die Spacergruppe PEG, eine Kohlenstoffkette, eine Kohlenstoffkette, die Schwefel, Stickstoff oder Sauerstoff in der Hauptkette einschließt, ein Polymer, ein Peptid oder irgendeine Kombonation davon einschließt.

53. Verwendung nach Anspruch 41, wobei eines oder mehrere Kohlenhydrat-Antigene an eine Verknüpfungsgruppe konjugiert sind.

54. Verwendung nach Anspruch 41, wobei ein oder mehrere Kohlenhydrat-Antigene, einzeln, an eine Spacergruppe konjugiert sind und eine oder mehrere Spacergruppen an eine Verknüpfungsgruppe konjugiert sind.

55. Verwendung nach Anspruch 54, wobei die Verknüpfungsgruppe wenigstens ein Schwefelatom, eine Carboxylatgruppe, eine Amidgruppe, eine Carbamatgruppe, eine Carbonatgruppe, eine Thiocarbamatgruppe, eine Thiocarbonatgruppe, eine Thioethergruppe, eine Succinimidgruppe, eine N-Hydroxysuccinimidgruppe oder irgendeine Kombination davon einschließt.

56. Verwendung nach Anspruch 41, wobei jedes der Kohlenhydrat-Antigene kovalent mit einem oder mehreren Schwefelatomen verknüpft ist.

57. Verwendung nach Anspruch 56, wobei wenigstens zwei der Schwefelatome aneinander gebunden sind.

58. Verwendung nach Anspruch 56, wobei die Kohlenhydrat-Antigene, einzeln, jeweils mit einem oder mehreren Schwefelatomen verknüpft sind.

59. Verwendung nach Anspruch 56, wobei wenigstens eines der Schwefelatome an wenigstens eines aus der Mehrzahl von Nanoteilchen unter Verwendung von NaBH₄ gebunden ist.

60. Verwendung nach Anspruch 59, wobei die Bindungen zwischen den Schwefelatomen und dem Nanoteilchen **dadurch gekennzeichnet sind, dass** sie kovalente Bindungen, ionische Bindungen, Wasserstoffbrückenbindungen, van-der-Waals-Bindungen oder irgendeine Kombination davon sind.

61. Verwendung nach Anspruch 41, wobei das Kohlenhydrat-Antigen ein Prognoseindikator, ein Marker für metastasierte Karzinomzellen, ein Adhäsionsmolekül, das bei Metastase involviert ist, oder irgendeine Kombination davon ist.

62. Verwendung nach Anspruch 41, wobei wenigstens eines aus der Mehrzahl der Nanoteilchen Goldatome, Silberatome, Platinatome, Rhodiumatome, Palladiumatome oder irgendeine Kombination davon einschließt.

63. Verwendung nach Anspruch 62, wobei die Nanoteilchen kolloidale Goldteilchen einschließen.

64. Verfahren nach Anspruch 1 oder 2, wobei die Molekulargewichte von wenigstens einem Teil der Antigen-Nanoteilchen-Konjugate im Bereich von etwa 1.000 Daltons bis etwa 1 Millionen Daltons liegt.

65. Verfahren nach Anspruch 64, wobei die Molekulargewichte von wenigstens einem Teil der Antigen-Nanoteilchen-Konjugate im Bereich von etwa 10.000 Daltons bis etwa 500.000 Daltons liegen.

66. Verwendung nach Anspruch 41, wobei wenigstens ein Teil der Antigen-Nanoteilchen-Konjugate, einzeln, von 2 bis etwa 1000 Kohlenhydrat-Antigene umfasst.

67. Verwendung nach Anspruch 41, wobei wenigstens ein Teil der Nanoteilchen von etwa 50 bis etwa 10.000 Atome umfasst.

68. Verwendung nach Anspruch 44, wobei wenigstens ein Teil der Nanoteilchen eine Abmessung im Bereich von etwa 0,5 nm bis etwa 100 nm besitzt.

69. Verwendung nach Anspruch 68, wobei wenigstens ein Teil der Nanoteilchen eine Abmessung im Bereich von etwa 1 nm bis etwa 10 nm besitzt.

70. Verwendung nach Anspruch 68, wobei wenigstens ein Teil der Nanoteilchen eine Kugelform umfasst und die Abmessung der Durchmesser des Nanoteilchens ist.

## Revendications

1. Méthode de préparation de conjugués de nanoparticules-antigènes, comprenant:
réaliser une nanoparticule; et
conjuguer une pluralité d'antigènes carbohydrates à la nanoparticule, où les antigènes carbohydrates comprennent un antigène TF.

2. Méthode selon la revendication 1, dans laquelle la pluralité d'antigènes carbohydrates comprend en outre l'antigène Tₙ, l'antigène Gb1, l'antigène GM₁, l'antigène GM₃, l'antigène Y de Lewis ou toute combinaison de ceux-ci.

3. Méthode selon la revendication 1 ou 2, dans laquelle une pluralité de nanoparticules sont prévues, et une pluralité d'antigènes carbohydrates sont conjuguées à au moins une portion des nanoparticules.

4. Méthode selon la revendication 3, dans laquelle une pluralité d'antigènes carbohydrates sont conjugués à chacune des nanoparticules.

5. Méthode selon la revendication 1 ou 2, dans laquelle les antigènes carbohydrates comprennent un groupe de liaison, et le groupe de liaison est conjugué à la nanoparticule.

6. Méthode selon la revendication 1 ou 2, dans laquelle la conjugaison comprend l'auto-assemblage des antigènes carbohydrates sur au moins une surface de la nanoparticule.

7. Méthode selon la revendication 1 ou 2, dans laquelle la nanoparticule comprend un ou plusieurs groupes de liaison de nanoparticules, et les antigènes carbohydrates sont conjugués aux groupes de liaison de nanoparticules.

8. Méthode selon la revendication 1 ou 2, dans laquelle un ou plusieurs groupes espaceurs lient les antigènes carbohydrates à la nanoparticule.

9. Méthode selon la revendication 8, dans laquelle un ou plusieurs groupes espaceurs précités comprennent PEG, une chaîne carbonée, une chaîne carbonée incluant du soufre, de l'azote ou de l'oxygène dans l'ossature, des polymères incluant le polyacrylamide, une chaîne peptidique constituée de toutes les unités de glycine ou d'alanine ou toute combinaison de ceux-ci.

10. Méthode selon la revendication 1 ou 2, comprenant en outre la purification des nanoparticules.

11. Méthode selon la revendication 10, dans laquelle ladite purification comprend la filtration, centrifugation électrophorèse, chromatographie, cristallisation ou toute combinaison de celles-ci.

12. Conjugué de nanoparticule-antigène comprenant une pluralité d'antigènes carbohydrates conjugués à la nanoparticule, où les antigènes comprennent l'antigène TF.

13. Conjugué de nanoparticule-antigène selon la revendication 12, dans lequel les antigènes comprennent en outre l'antigène Tₙ, l'antigène Gb1, l'antigène GM₁, l'antigène GM₃, l'antigène Y de Lewis ou toute combinaison de ceux-ci.

14. Conjugué de nanoparticule-antigène selon la revendication 12 ou 13, dans lequel la pluralité d'antigènes carbohydrates sont identiques entre eux.

15. Conjugué de nanoparticule-antigène selon la revendication 12 ou 13, dans lequel au moins une portion des antigènes carbohydrates sont affichés au système immunitaire dans des carcinomes humains durant la croissance et la progression de la tumeur.

16. Conjugué de nanoparticule-antigène selon la revendication 12 ou 13, dans lequel les antigènes carbohydrates sont aptes à induire une expression surfaçique de galectine-3 dans des cellules endothéliales.

17. Conjugué de nanoparticule-antigène selon la revendication 12 ou 13, dans lequel au moins un de la pluralité d'antigènes carbohydrates associés à la tumeur est conjugué à un groupe espaceur, et le groupe espaceur est conjugué à la nanoparticule.

18. Conjugué de nanoparticule-antigène selon la revendication 12 ou 13, dans lequel le groupe espaceur comprend du PEG, une chaîne carbonée, une chaîne carbonée incluant du soufre, de l'azote ou de l'oxygène dans l'ossature, un polymère, un peptide ou toute combinaison de ceux-ci.

19. Conjugué de nanoparticule-antigène selon la revendication 12 ou 13, dans lequel un ou plusieurs antigènes carbohydrates sont conjugués à un ou plusieurs groupes de liaison, et les groupes de liaison sont conjugués à la nanoparticule.

20. Conjugué de nanoparticule-antigène selon la revendication 12 ou 13, dans lequel un ou plusieurs antigènes carbohydrates sont conjugués à un ou plusieurs groupes espaceurs, les groupes espaceurs sont conjugués à un ou plusieurs groupes de liaison, et un ou plusieurs groupes de liaison précités sont conjugués à la nanoparticule.

21. Méthode selon la revendication 4 ou conjugué de nanoparticule-antigène selon la revendication 19 ou 20, dans lequel les groupes de liaison comprennent au moins un atome de soufre, groupe carboxylate, groupe amide, groupe carbamate, groupe carbonate, groupe thiocarbamate, groupe thiocarbonate, groupe thioéther, groupe succinimide, groupe n-hydroxy succinimide ou toute combinaison de ceux-ci.

22. Conjugué de nanoparticule-antigène selon la revendication 12 ou 13, dans lequel chacun des antigènes carbohydrates sont liés de manière covalente à un ou plusieurs atomes de soufre.

23. Conjugué de nanoparticule-antigène selon la revendication 12 ou 13, dans lequel les antigènes carbohydrates sont chacun liés, individuellement, à un ou plusieurs atomes de soufre.

24. Conjugué de nanoparticule-antigène selon la revendication 12 ou 13, dans lequel chacun des antigènes carbohydrates comprend un ou plusieurs acides aminés.

25. Conjugué de nanoparticule-antigène selon la revendication 22, dans lequel au moins deux atomes de soufre sont liés de manière covalente l'un à l'autre.

26. Conjugué de nanoparticule-antigène selon la revendication 22, dans lequel au moins un des atomes de soufre est lié à la nanoparticule.

27. Conjugué de nanoparticule-antigène selon la revendication 26, dans lequel les liaisons entre les atomes de soufre et la nanoparticule comprennent des liaisons covalentes, des liaisons hydrogènes, des liaisons ioniques, des liaisons van der Waals, ou toute combinaison de celles-ci.

28. Conjugué de nanoparticule-antigène selon la revendication 12 ou 13, dans lequel les antigènes carbohydrates comprennent une disaccharide.

29. Conjugué de nanoparticule-antigène selon la revendication 28, dans lequel ledit disaccharide comprend au moins un groupe de sucre amino.

30. Conjugué de nanoparticule-antigène selon la revendication 12 ou 13, dans lequel au moins un de la pluralité d'antigènes carbohydrates est un indicateur de pronostic pour le cancer, un marqueur de cellules de carcinome métastasées, une molécule d'adhésion impliquée dans la métastase ou toute combinaison de ceux-ci.

31. Conjugué de nanoparticule-antigène selon la revendication 12 ou 13, dans lequel la nanoparticule comprend des atomes d'or, atomes d'argent, atomes de platine, atomes de rhodium, atomes de palladium ou toute combinaison de ceux-ci.

32. Conjugué de nanoparticule-antigène selon la revendication 31, dans lequel la nanoparticule est dérivée d'or colloïdal.

33. Conjugué de nanoparticule-antigène selon la revendication 12 ou 13, dans lequel le conjugué de nanoparticule-antigène a un poids moléculaire dans la plage d'environ 1000 daltons à environ 1 million de Daltons.

34. Conjugué de nanoparticule-antigène selon la revendication 12 ou 13, dans lequel le nombre d'antigènes carbohydrates conjugués à la nanoparticule est dans la plage d'environ 2 à environ 1000.

35. Conjugué de nanoparticule-antigène selon la revendication 12 ou 13, dans lequel la nanoparticule à environ 50 à environ 10 000 atomes.

36. Conjugué nanoparticule-antigène selon la revendication 12 ou 13, dans lequel la nanoparticule a une dimension dans la plage d'environ 0,5 nm à environ 100 nm.

37. Conjugué de nanoparticule-antigène selon la revendication 36, dans lequel la nanoparticule a une dimension dans la plage d'environ 1 nm à environ 10 nm.

38. Conjugué de nanoparticule-antigène selon la revendication 36, dans lequel la nanoparticule a une forme sphéroïde, et le diamètre de la nanoparticule est dans la plage d'environ 1 nm à environ 10 nm.

39. Conjugué de nanoparticule-antigène selon la revendication 36, dans lequel la nanoparticule a une forme icosaédrique, cubique, rhombique, hexagonale ou de fullérène.

40. Conjugué de nanoparticule-antigène selon l'une quelconque des revendications 12-39 pour utilisation en thérapie.

41. Utilisation du conjugué de nanoparticule-antigène selon l'une quelconque des revendications 12-39 pour la fabrication d'un médicament pour inhiber la métastase de cellules de carcinome chez un mammifère.

42. Utilisation selon la revendication 41, où le mammifère est un être humain.

43. Utilisation selon la revendication 42, où un cancer est diagnostiqué chez l'être humain.

44. Utilisation selon la revendication 43, où le cancer est un cancer du sein.

45. Utilisation selon la revendication 44, où une métastase à des cellules pulmonaires est inhibée.

46. Utilisation selon la revendication 41, comprenant en outre le retrait de cellules tumorales dudit mammifère.

47. Utilisation selon la revendication 46, où les cellules tumorales sont retirées dudit mammifère avant l'administration de la quantité thérapeutiquement efficace des conjugués de nanoparticule-antigène.

48. Utilisation selon la revendication 41, où les antigènes carbohydrates sont affichés au système immunitaire dans des carcinomes humains durant la croissance et la progression de la tumeur.

49. Utilisation selon la revendication 41, où les antigènes carbohydrates sont aptes à induire une expression surfaçique de galectine-3 dans des cellules endothéliales.

50. Utilisation selon la revendication 41, où les antigènes carbohydrates sont conjugués à une surface extérieure de ladite nanoparticule.

51. Utilisation selon la revendication 41, dans laquelle au moins un des antigènes carbohydrates associés à la tumeur est conjugué, individuellement, à au moins un groupe espaceur, et le au moins un groupe espaceur est conjugué à la nanoparticule.

52. Utilisation selon la revendication 51, où le groupe espaceur comprend PEG, une chaîne carbonnée, une chaîne carbonnée incluant du soufre, de l'azote ou de l'oxygène dans l'ossature, un polymère, un peptide ou toute combinaison de ceux-ci.

53. Utilisation selon la revendication 41, où un ou plusieurs antigènes carbohydrates sont conjugués à un groupe de liaison.

54. Utilisation selon la revendication 41, où un ou plusieurs antigènes carbohydrates sont conjugués, individuellement, à un groupe espaceur, et un ou plusieurs groupes espaceurs sont conjugués à un groupe de liaison.

55. Utilisation selon la revendication 54, où le groupe de liaison comprend au moins un atome de soufre, groupe carboxylate, groupe amide, groupe carbamate, groupe carbonate, groupe thiocarbamate, groupe thiocarbonate, groupe thioéther, groupe succinimide, groupe n-hydroxy succinimide ou toute combinaison de ceux-ci.

56. Utilisation selon la revendication 41, où chacun des antigènes carbohydrates est lié de manière covalente à un ou plusieurs atomes de soufre.

57. Utilisation selon la revendication 56, où au moins deux des atomes de soufre sont liés l'un à l'autre.

58. Utilisation selon la revendication 56, où les antigènes carbohydrates sont chacun liés, individuellement, à un ou plusieurs atomes de soufre.

59. Utilisation selon la revendication 56, où au moins un des atomes de soufre est lié à au moins une de la pluralité de nanoparticules en utilisant NaBH₄.

60. Utilisation selon la revendication 59, où les liaisons entre les atomes de soufre et la nanoparticule sont caractérisées comme étant des liaisons covalentes, liaisons ioniques, liaisons d'hydrogène, liaisons van der Waals ou toute combinaison de celles-ci.

61. Utilisation selon la revendication 41, où les antigènes carbohydrates sont un indicateur de pronostic, un marqueur de cellules de carcinome métastasées, une molécule d'adhésion impliquée dans la métastase ou toute combinaison de ceux-ci.

62. Utilisation selon la revendication 41, où au moins une de la pluralité de nanoparticules comprend des atomes d'or, atomes d'argent, atomes de platine, atomes de rhodium, atomes de palladium ou toute combinaison de ceux-ci.

63. Utilisation selon la revendication 62, où les nanoparticules comprennent des particules d'or colloïdales.

64. Méthode selon la revendication 1 ou 2, où les poids moléculaires d'au moins une portion des conjugués de nanoparticule-antigène est dans la plage d'environ 1000 Daltons à environ 1 million de Daltons.

65. Méthode selon la revendication 64, où les poids moléculaires d'au moins une portion des conjugués de nanoparticule-antigène sont dans la plage d'environ 10 000 Daltons à environ 500 000 Daltons.

66. Utilisation selon la revendication 41, où au moins une portion des conjugués de nanoparticule-antigène comprennent, individuellement, de 2 à environ 1000 antigènes carbohydrates.

67. Utilisation selon la revendication 41, où au moins une portion des nanoparticules comprennent environ 50 à environ 10 000 atomes.

68. Utilisation selon la revendication 44, où au moins une portion des nanoparticules a une dimension dans la plage d'environ 0,5 nm à environ 100 nm.

69. Utilisation selon la revendication 68, où au moins une portion des nanoparticules a une dimension dans la plage d'environ 1 nm à environ 10 nm.

70. Utilisation selon la revendication 68, où au moins une portion des nanoparticules comprend une forme sphéroïde, et la dimension est le diamètre de la nanoparticule.
